(19) Europäisches Patentamt
European Patent Office
Office européen des brevets

(11) EP 1 315 806 B1

(12) EUROPEAN PATENT SPECIFICATION

(45) Date of publication and mention of the grant of the patent:
**28.12.2005 Bulletin 2005/52**

(21) Application number: **01960178.0**

(22) Date of filing: **17.07.2001**

(51) Int Cl.[7]: **C12N 15/10**, C12N 15/62, C12Q 1/37, C07K 1/04

(86) International application number:
**PCT/DK2001/000503**

(87) International publication number:
**WO 2002/018588 (07.03.2002 Gazette 2002/10)**

# (54) METHOD FOR SCREENING HIGHLY ACTIVE PROTEASES AND INHIBITORS

VERFAHREN ZUM SCREENING VON HOCHAKTIVEN PROTEASEN UND HEMMSTOFFEN

PROCEDE DE CRIBLAGE DE PROTEASES HAUTEMENT ACTIVES ET LEURS INHIBITEURS

(84) Designated Contracting States:
**AT BE CH CY DE DK ES FI FR GB GR IE IT LI LU MC NL PT SE TR**

(30) Priority: **29.08.2000 DK 200001273**

(43) Date of publication of application:
**04.06.2003 Bulletin 2003/23**

(73) Proprietor: **Novozymes A/S**
**2880 Bagsvaerd (DK)**

(72) Inventors:
- **PEDERSEN, Poul, Erik**
  **DK-2860 Soborg (DK)**
- **NÖRREGAARD-MADSEN, Mads**
  **DK-3460 Birkeröd (DK)**

(56) References cited:
**WO-A1-00/40745**
**WO-A1-93/01305**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 315 806 B1

Printed by Jouve, 75001 PARIS (FR)

## Description

### Field of the Invention

This invention relates to a method of industrial screening for proteases from a protease gene library. The proteases are produced *in vivo* as proteolytically inactive complexes, each complex comprising two interacting parts, a protease part and a protease inhibitor part. The inhibitor part effectively minimizes proteolytic activity of the protease part of the complex during production and/or recovery steps, whereafter the complex may be dissociated to assay the proteolytic activity of the protease part. The complex may also simply be recovered and used directly in a relevant application assay e.g. in a washing detergent assay where the inhibitor part dissociates from the protease in the dilute detergent while the assay is being performed.

### Background of the Invention

In the detergent industry enzymes have for more than 30 years been implemented in washing formulations. Enzymes used in such formulations comprise proteases, lipases, amylases, cellulases, as well as other enzymes, or mixtures thereof. Commercially the most important enzymes are proteases such as Alcalase® (Novo Nordisk), Kannase® (Novo Nordisk) Savinase® (Novo Nordisk), or Esperase® (Novo Nordisk).

An increasing number of commercially used proteases are protein engineered variants of naturally occurring wild type proteases, e.g. Durazym® (Novo Nordisk), Relase® (Novo Nordisk), Maxapem® (Gist-Brocades), Purafect® (Genencor).

It has been described to treat protease with added protease inhibitors in WO 93/20175; WO 93/13125; WO 92/05239; WO 93/17086 (Novo Nordisk) or to fuse a protease covalently with a Streptomyces SSI protease inhibitor in WO 00/01831 (Procter & Gamble); and WO 98/13483 (Procter & Gamble).

However, even though a number of useful protease variants have been described, there is still a need for new proteases or protease variants with new and/or improved properties especially with improved activity under specific conditions.

### Summary of the Invention

The problem to be solved by the present invention is how to screen for a gene encoding a protease or a protease inhibitor from libraries of such. The solution to the problem relies on reversibly inhibiting the proteolytic activity of a protease by providing an inhibitor *in vivo.* The protease and the inhibitor are co-synthesized in a host cell which produces a proteolytically inactive complex of the protease and the inhibitor.

If the screening is for a protease gene, then a protease gene library is co-expressed in a host cell with a specific inhibitor, and if the screening is for an inhibitor gene, then an inhibitor gene library is co-expressed with a specific protease.

The method relates to an improvement in two separate but inter-related articles, a protease and a protease inhibitor, that may be produced separately, but each are indispensible for the formation of the proteolytically inactive protease-inhibitor complex on which the method of the invention relies.

The method of the invention is especially relevant when screening for a protease with an increased proteolytic activity when compared to the other proteases of the library. Such a highly active protease may be difficult to identify in conventional screening assays, as highly active proteases are prone to degradation by autoproteolysis already during production, sometimes to the point where they may be virtually undetectable after fermentation and recovery steps.

As already mentioned it is desirable to minimize autoproteolysis during production and recovery of industrial proteases. Once a protease has been selected for large-scale production it can be advantageous to produce the protease as a proteolytically inactive complex with a protease inhibitor bound to the protease. The present invention also provides an effective method of screening for en improved protease inhibitor, where a certain protease is co-synthesized with the protease inhibitor library. An inhibitor of interest from the library is then chosen according to the method e.g. on the basis of improved protease binding/dissociating abilities of the inhibitor under certain conditions of interest.

The method of the invention utilizes the results shown herein to assay proteases under conditions of interest, in order to determine their true proteolytic activity under these conditions. The assay result makes a qualified selection possible of a gene encoding a protease of interest from a library of protease genes.

As mentioned above, it has been suggested to express the Streptomyces subtilisin inhibitor (SSI) as a fusion protein with a protease, it was reported that the covalently linked protease-SSI fusion exhibited no protease activity and it was sketchily described how to determine the protease activity of the fusion protein under normal washing conditions to see whether protease activity would be restored, however no data were given as to whether this was truly the case, and the assertion of reversability of the protease inhibition was not documented.

We have previously identified a number of barley chymotrypsin inhibitor CI-2A variants having a reduced constant of interaction, $K_i$, for subtilisin 309 when compared to the wild type CI-2A *(vide supra).* The present inventors have now constructed an *in frame* fusion polynucleotide sequences of a gene encoding a protease with a sequence encoding a small peptide linker and with a gene encoding the wild type barley chymotrypsin inhibitor CI-2A or a variant of the CI-2A inhibitor (M59P) that we previously identified. We expressed these fusion genes and tested the resulting protein products.

Expression of the fusion genes gave rise to the production of protease-inhibitor complexes, and treatment of the complexes with dilute detergent to dissociate them revealed surprisingly that the complexes were not covalently linked, they turned out to be composed of a protease part and an inhibitor part (see examples below).

The protease complex with the wild type CI-2A inhibitor turned out to be very tightly bound and it did not dissociate in a dilute detergent but rather required higher detergent concentrations, whereas the protease CI-2A(M59P) complex dissociated completely in a dilute detergent (examples below), a finding which corresponds well with our previous observations on the interaction constant, $K_i$, of the respective CI-2A inhibitor variants *(supra).*

The fact that the two parts of the protease-inhibitor complex are not covalently linked allows highly active proteases to be produced without reduction in yield due to autoproteolysis during fermentation and recovery steps, whereupon a simple dissociation step will allow subsequent recovery of the active protease without the inhibitor, and with minimal loss of activity; the whole complex may even be recovered and kept inactive until application where the complex dissociates and the protease becomes active e.g. in dilute detergent washing.

Presently we have shown that the activity from the subtilisin part of a CI-2A(M59P) complex that was *in vivo* synthesized, was indistinguishable from the activity of the pure subtilisin under test washing conditions (below).

Accordingly the invention relates to a method of screening a protease gene library for a gene encoding a protease of interest, the method comprising the steps of:

a) constructing a host cell comprising a first gene of the protease gene library and a second gene encoding a protease inhibitor;
b) cultivating the host cell, wherein the cell expresses the first and the second genes to produce a complex of a protease and the inhibitor;
c) dissociating the inhibitor from the complex; and
d) selecting the protease of interest and isolating the encoding gene.

Further aspects which are not part of the invention as defined by the appended claims include the following:

A second aspect relates to a method of screening a protease inhibitor gene library for a gene encoding a protease inhibitor of interest, the method comprising the steps of:

a) constructing a host cell comprising a first gene encoding a protease and a second gene of the protease inhibitor library;
b) cultivating the host cell, wherein the cell expresses the first and the second genes to produce a complex of the protease and an inhibitor;
c) dissociating the inhibitor from the complex; and
d) selecting the inhibitor of interest and isolating the encoding gene.

Various standard means exist in the art, whereby the degeneracy of the genetic code can be utilized to change a polynucleotide sequence or codon optimize a sequence in such a manner that the amino acid sequence that is encoded remains the same. The method is intended to be used also for codon usage variants of the genes described, in any kind of polynucleotide construct, non-limiting examples of which could be plasmids, integration cassettes, and transposons.

A third aspect relates to a polynucleotide construct comprising a protease encoding gene obtainable by the method defined in the first aspect, preferably the construct further comprises a gene encoding a protease inhibitor.

A fourth aspect relates to a protease encoded by a gene obtainable by the method defined in the first aspect.

A fifth aspect relates to a polynucleotide construct comprising a protease inhibitor gene obtainable by a method as defined in the second aspect, preferably the construct further comprises a gene encoding a protease.

A sixth aspect relates to a protease inhibitor encoded by a gene obtainable by the method defined in the second aspect.

For industrial production purposes it is necessary to cultivate host cells comprising a polynucleotide construct as defined in the previous aspect in order to produce a protein complex, or selected protease, as defined in the first aspect. A preferred host cell genus of the industrial enzyme manufacturers is *Bacillus,* especially cells of the species *Bacillus alkalophilus, Bacillus amyloliquefaciens, Bacillus brevis, Bacillus circulans, Bacillus coagulans, Bacillus lautus,*

*Bacillus lentus, Bacillus licheniformis, Bacillus megaterium, Bacillus stearothermophilus, Bacillus subtilis*, and *Bacillus thuringiensis*.

The final aspect relates to a host cell comprising a polynucleotide construct as defined in the third or fifth aspects, preferably the host cell is of a *Bacillus* species, and more preferably the host cell is a *B. subtilis, B. clausii,* or *B. licheniformis* cell.

An integrate part of the invention is that the complex has to be placed under conditions that will dissociate the inhibitor part from the protease part, such conditions are typically found whereever detergents are applied, in cleaning compositions and/or additives of any kind.

## Definitions

General techniques

In general standard procedures for cloning of genes and introducing insertions (random and/or site directed) into said genes may be used in order to obtain a subtilase enzyme of the invention. For further description of suitable techniques reference is made to Examples herein *(vide infra)* and (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990); and WO 96/34946.

Isolated nucleic acid sequence

The term "isolated nucleic acid sequence" as used herein refers to a nucleic acid sequence, which has been isolated and purified and is thus in a form suitable for use within genetically engineered protein production systems. Such isolated molecules may be those that are separated from their natural environment and include cDNA and genomic clones as well as nucleic acid sequences derived from DNA shuffling experiments as described in US 5,605,793; US 5,830,721; US 5,811,238; US 5,834,252; US 5,928,905; US 5,837,458; WO 98/41653; WO 98/41623; WO 98/41622; or WO 95/22625, or from site-directed mutagenisis experiments. Isolated nucleic acid sequences of the present invention are free of other genes with which they are ordinarily associated, but may include 5' and 3' untranslated regions such as promoters and terminators. The identification of associated regions will be evident to one of ordinary skill in the art (see for example, Dynan and Tijan, Nature 316:774-78, 1985). The term "isolated nucleic acid sequence" may alternatively be termed "isolated DNA sequence, "cloned nucleic acid sequence" or "cloned DNA sequence".

Gene library

The term "gene library" as used herein refers to a collection of nucleic acid sequences, which have been isolated or cloned. The library may include cDNA and genomic clones as well as artificial sequences, mutagenized or shuffled sequences as described above. A gene library preferably comprises over $10^6$ clones, more preferably over $10^8$ clones, even more preferably over $10^{10}$ clones, still more preferably over $10^{12}$ dones, and most preferably over $10^{14}$ clones.

Isolated protein

When applied to a protein, the term "isolated" indicates that the protein has been removed from its native environment. In a preferred form, the isolated protein is substantially free of other proteins, particularly other homologous proteins (*i.e*. "homologous impurities" (see below)). An isolated protein is more than 10% pure, preferably more than 20% pure, more preferably more than 30% pure, as determined by SDS-PAGE. Further it is preferred to provide the protein in a highly purified form, i.e., more than 40% pure, more than 60% pure, more than 80% pure, more preferably more than 95% pure, and most preferably more than 99% pure, as determined by SDS-PAGE. The term "isolated protein" may alternatively be termed "purified protein".

Serine proteases

A serine protease is an enzyme which catalyzes the hydrolysis of peptide bonds, and in which there is an essential serine residue at the active site (White, Handler and Smith, 1973 "*Principles of Biochemistry*," Fifth Edition, McGraw-Hill Book Company, NY, pp. 271-272).

The bacterial serine proteases have molecular weights in the 20,000 to 45,000 Dalton range. They are inhibited by diisopropylfluorophosphate. They hydrolyze simple terminal esters and are similar in activity to eukaryotic chymotrypsin, also a serine protease. A more narrow term, alkaline protease, covering a sub-group, reflects the high pH optimum of some of the serine proteases, from pH 9.0 to 11.0 (for review, see Priest (1977) *Bacteriological Rev.* 41 711-753).

Subtilases

A sub-group of the serine proteases tentatively designated subtilases has been proposed by Siezen *et al*., *Protein Engng.* 4 (1991) 719-737 and Siezen et al. *Protein Science* 6 (1997) 501-523. They are defined by homology analysis of more than 170 amino acid sequences of serine proteases previously referred to as subtilisin-like proteases. A subtilisin was previously often defined as a serine protease produced by Gram-positive bacteria or fungi, and according to Siezen *et al*. now is a subgroup of the subtilases. A wide variety of subtilases have been identified, and the amino acid sequence of a number of subtilases has been determined. For a more detailed description of such subtilases and their amino acid sequences reference is made to Siezen *et al*.(1997).

One subgroup of the subtilases, I-S1 or "true" subtilisins, comprises the "classical" subtilisins, such as subtilisin 168 (BSS168), subtilisin BPN', subtilisin Carlsberg (Alcalase®, Novo Nordisk A/S), and subtilisin DY (BSSDY).

A further subgroup of the subtilases, I-S2 or high alkaline subtilisins, is recognized by Siezen *et al. (supra).* Sub-group I-S2 proteases are described as highly alkaline subtilisins and comprises enzymes such as subtilisin PB92 (BAALKP) (Maxacal®, Gist-Brocades NV), subtilisin 309 (Savinase®, Novo Nordisk A/S), subtilisin 147 (BLS147) (Esperase®, Novo Nordisk A/S), and alkaline elastase YaB (BSEYAB).

Parent subtilase

The term "parent subtilase" describes a subtilase defined according to Siezen et al. (1991 and 1997), for further details see description of "Subtilases" above. A parent subtilase may also be a subtilase isolated from a natural source, alternatively the term "parent subtilase" may be termed "wild type subtilase".

Modification(s) of a subtilase

The term "modification(s)" of a subtilase used herein is defined to include chemical modification of a subtilase as well as genetic manipulation of the DNA encoding a subtilase. The modification(s) can be replacement(s) of the amino acid side chain(s), substitution(s), deletion(s) and/or insertions in or at the amino acid(s) of interest.

In this specification and the claims protein variants to be used or contemplated to be used in the present invention are described using the following nomenclatures for ease of reference:

Original amino acid(s); position(s); substituted amino acid(s)

According to this the substitution of Glutamic acid for glycine in position 195 is indicated as:

Gly 195 Glu or G195E A deletion of glycine in the same position is indicated as:
Gly 195 * or G195* Insertion of an additional amino acid residue such as lysine is shown as:
Gly 195 GlyLys or G195GK An insertion of an aspartic acid in position 36 is indicated as:
* 36 Asp or *36D Multiple variants are separated by pluses, i.e.:
Arg 170 Tyr + Gly 195 Glu or R170Y+G195E

representing a multiple variant "mutated" in positions 170 and 195 substituting tyrosine and glutamic acid for arginine and glycine, respectively.

In the context of this invention, the term subtilase variant or mutated subtilase means a subtilase that has been derived from a parent enzyme, the parent gene having been mutated in order to produce a mutant gene from which said mutated subtilase protease is produced when expressed in a suitable host. Analogously, the mutant gene may also be derived from a parent gene produced by DNA shuffling techniques as known in the art.

The present invention comprises any one or more modifications to the amino acid sequence of the parent subtilase. Especially combinations with other modifications known in the art to provide improved properties to the enzyme are envisaged. The art describes a number of subtilase variants with different improved properties and a number of those are mentioned in the "Background of the invention" section herein *(vide supra).*

Such combinations comprise the positions: 222 (improve oxidation stability), 218 (improves thermal stability), substitutions in the Ca-binding sites stabilising the enzyme, e.g. position 76, and many other apparent from the prior art.

In further embodiments a subtilase variant of the invention may advantageously be combined with one or more modification(s) in any of the positions: 27, 36, 57, 76, 97, 101, 104, 120, 123, 167, 170, 195, 206, 218, 222, 224, 235, 252, 255, 259 and 274.

Specifically the following subtilisin 309 and subtilisin BAPB92 variants are considered appropriate for combination:

K27R, *36D, S57P, N76D, G97N, S101G, S103A, V104A, V104N, V104Y, V104I, H120D, N123S, G159D, Y167A, Y167I, R170S, R170L, R170N, A194P, G195E, Q206E, Y217L, N218S, M222S, M222A, T224S, A232V, K235L, Q236H, Q245R, N248D, N252K, and T274A.

Furthermore variants comprising any of the variants Y167A+R170S+A194P, V104N+S101G, K27R+V104Y+N123S+T274A, N76D+S103A+V104I, or S101G+S103A+V104I+G159D+A232V+Q236H+Q245R+N248D+N252K, or other combinations of these mutations (V104N, S101G, K27R, V104Y, N123S, T274A, N76D, S103A, V104I, G159D, A232V, Q236H, Q245R, N248D, N252K), in combination with any one or more of the modification(s) mentioned above exhibit improved properties.

Even further subtilase variants of the main aspect(s) of the invention are preferably combined with one or more modification(s) in any of the positions 129, ,131, 133 and 194, preferably as 129K, 131 H, 133P, 133D and 194P modifications, and most preferably as P129K, P131H, A133P, A133D and A194P modifications. Any of those modification(s) may give a higher expression level of a subtilase variant of the invention.

Many methods for cloning a subtilase of the invention and for introducing mutations into genes *(e.g.* subtilase genes) are well known in the art. In general standard procedures for cloning of genes and introducing mutations (random and/or site directed) into said genes may be used in order to obtain a subtilase variant of the invention. For further description of suitable techniques reference is made to working examples herein (*vide infra)* and (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990); and WO 96/34946.

## Numbering of amino acid positions/residues

If nothing else is mentioned, the amino acid numbering used herein corresponds to that of the subtilase BPN' (BASBPN) sequence. For further description of the BPN' sequence see Siezen *et al., Protein Engng.* 4 (1991), p. 719-737. A frame of reference is defined by aligning an isolated or a parent enzyme with subtilisin BPN' (BASBPN).

An alignment can be obtained by using the GAP routine of the GCG program package version 9.1 to number the subtilases using the following parameters: gap creation penalty = 8, and gap extension penalty = 8, and all other parameters kept at their default values.

Alignments of sequences and calculation of identity scores can be done using a full Smith-Waterman alignment, useful for both protein and DNA alignments. The default scoring matrices BLOSUM50 and the identity matrix are used for protein and DNA alignments respectively. The penalty for the first residue in a gap is -12 for proteins and -16 for DNA, while the penalty for additional residues in a gap is -2 for proteins and -4 for DNA. Align is from the fasta package version v20u6 (W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA" Methods in Enzymology 183:63-98).

Another method is to use known recognized alignments between subtilases, such as the alignment indicated in WO 91/00345. In most cases the differences will not be of any importance.

## Homologous subtilase sequences

In the present context the homology between two amino acid sequences is described by the parameter "identity".

In order to determine the degree of identity between two subtilases the GAP routine of the GCG package version 9.1 can be applied *(infra)* using the same settings. The output from the routine is besides the amino acid alignment the calculation of the "Percent Identity" between the two sequences.

Based on this description it is routine for a person skilled in the art to identify suitable homologous subtilases and corresponding homologous active site loop regions, which can be modified according to the invention.

## Inhibitor

The proteinaceous protease inhibitors of the invention are typically long peptides (often over 28 amino acids), which bind to the active site of a protease and inhibit its activity. They have been classified into several Families (I-IX) based on primary amino acid sequence homologies (Laskowski, M., Jr., and I. Kato, "Protein inhibitors of proteinases", *Ann. Rev. Biochem.* (1980) 49:593-626). The inhibitors may be derived from the known inhibitors of Family VI, non-limiting examples of which are *Streptomyces* Subtilase Inhibitors (SSI), Eglin C inhibitors, pumpkin trypsin inhibitors (CMTI), or barley chymotrypsin inhibitors (CI), all of which are well characterized in the art. They may e.g. be derived from barley subtilisin inhibitor CI-1 or CI-2A.

Inhibitors of this family are known to strongly inhibit the subtilisins commonly used in detergents, with inhibitor constants of interaction generally below $10^{-10}$ M. We have found that by using these inhibitors to stabilize a protease in a detergent, the protease is so strongly bound that very little protease activity is released when the detergent is diluted for use in washing, and the protease remains almost completely inactive. We have therefore identified a need for a modified inhibitor with weaker binding to the protease.

We have previously found that the protease-inhibitor binding of CI-2A can be suitably weakened by substituting the P1 residue with Pro (M59P) (WO 93/20175; Novo Nordisk) and we've identified a number of other modifications of the barley CI-2A inhibitor resulting in a higher constant of interaction, $K_i$ (WO 92/05239 and WO 93/17086; Novo Nordisk).

Starting from the reactive site, amino acids positions of the inhibitors are numbered P1, P2 etc. in the direction of the N-terminal; and P'1, P'2 etc. towards the C-terminal according to Schechter and Berger (1967; Biochem Biophys Res Commun. 27:157-162). The following shows the amino acid sequence in the binding region of CI-2A as well as the modifications identified previously which improve the properties of the inhibitor for the present invention:

| | P6 | P5 | P4 | P3 | P2 | P1 | P'1 | P'2 | P'3 |
|---|---|---|---|---|---|---|---|---|---|
| **CI-2A** | Gly | Thr | Ile | Val | Thr | Met | Glu | Tyr | Arg |

**Modifications:**

P6: Ala, Glu, Tyr, Pro or Lys
P5: Gly, Val, Leu, Glu, Ile or Pro
P4: Val, Pro, Trp, Ser, Glu, Gly, Lys or Arg
P3: Tyr, Glu, Ala, Arg, Pro, Ser, Lys, or Trp
P2: Ser, Lys, Arg, Pro, Glu, Val, Tyr, Trp, Ile, Gly or Ala
P1: Arg, Tyr, Pro, Trp, Glu, Val, Ser, Lys, Asp, Ile, Gly, or Ala
P'1: Gln, Ser, Thr, Ile, Lys, Asn, or Pro
P'2: Val, Glu, Arg, Pro, Gly or Trp
P'3: Glu, Gln, Asn, Val, Phe, Ile, Thr or Tyr.

Protein inhibitor complex

A protease inhibitor complex comprises at least a protease part and a protease inhibitor part, where the inhibitor binds to the active site of a protease and inhibit its activity. The protease and the inhibitor may be covalently linked but preferably they are not covalently linked.

Fusion polynucleotide

A fusion polynucleotide of the invention has the meaning generally recognized in the art; a polynucleotide sequence that comprises sequences originally encoding two or more parent proteins or variants thereof, where the coding sequences have been fused to form a single open reading frame *i.e.* the sequences are fused *in frame.* In the fusion polynucleotide, additional nucleotides may have been added to the sequences encoding the parent proteins both 5'- and 3'-terminally to form linkers or spacers between or flanking the parent sequences in the sequence of the fusion polynucleotide. N-terminal leader encoding sequences may also have been added such as pro-, pre-pro-, or secretion signals.

Nucleic acid sequences

The present invention also relates to an isolated nucleic acid sequence, which encodes a protease inhibitor complex of the present invention.

The techniques used to isolate or clone a nucleic acid sequence encoding a polypeptide are known in the art and include isolation from genomic DNA, preparation from cDNA, or a combination thereof. The cloning of the nucleic acid sequences of the present invention from such genomic DNA can be effected, *e.g.*, by using the well-known polymerase chain reaction (PCR) or antibody screening of expression libraries to detect cloned DNA fragments with shared structural features. See, *e.g.*, Innis *et al.,* 1990, *PCR: A Guide to Methods and Application,* Academic Press, New York. Other nucleic acid amplification procedures such as ligase chain reaction (LCR), ligated activated transcription (LAT) and nucleic acid sequence-based amplification (NASBA) may be used.

An isolated nucleic acid sequence can, for example, be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the subtilase, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

For purposes of the present invention, the degree of identity between two nucleic acid sequences is determined is described above.

Modification of a nucleic acid sequence encoding a subtilase of the present invention may be necessary for the synthesis of subtilases substantially similar to the subtilase. The term "substantially similar" to the subtilase refers to non-naturally occurring forms of the subtilase. These subtilases may differ in some engineered way from the subtilase isolated from its native source, e.g., variants that differ in specific activity, thermostability, pH optimum, or the like. For a general description of nucleotide substitution, see, *e.g.*, Ford *et al.,* 1991, *Protein Expression and Purification* 2: 95-107.

It will be apparent to those skilled in the art that such substitutions can be made outside the regions critical to the function of the molecule and still result in an active subtilase. Amino acid residues essential to the activity of the polypeptide encoded by the isolated nucleic acid sequence of the invention, and therefore preferably not subject to substitution, may be identified according to procedures known in the art, such as site-directed mutagenesis or alanine-scanning mutagenesis (see, e.g., Cunningham and Wells, 1989, *Science* 244: 1081-1085). In the latter technique, mutations are introduced at every positively charged residue in the molecule, and the resultant mutant molecules are tested for proteolytic activity to identify amino acid residues that are critical to the activity of the molecule. Sites of substrate-enzyme interaction can also be determined by analysis of the three-dimensional structure as determined by such techniques as nuclear magnetic resonance analysis, crystallography or photoaffinity labelling (see, e.g., de Vos *et al.,* 1992, *Science* 255: 306-312; Smith *et al.,* 1992, *Journal of Molecular Biology* 224: 899-904; Wlodaver *et al.,* 1992, *FEBS Letters* 309: 59-64).

Nucleic acid constructs

The present invention also relates to nucleic acid constructs comprising a nucleic acid sequence of the present invention operably linked to one or more control sequences capable of directing the expression of the polypeptide in a suitable host cell.

An isolated nucleic acid sequence encoding a protease inhibitor complex of the present invention may be manipulated in a variety of ways to provide for expression of the subtilase. Manipulation of the nucleic acid sequence prior to its insertion into a vector may be desirable or necessary depending on the expression vector. The techniques for modifying nucleic acid sequences utilizing recombinant DNA methods are well known in the art.

The control sequences include all components that are necessary or advantageous for the expression of a subtilase of the present invention. Each control sequence may be native or foreign to the nucleic acid sequence encoding the subtilase. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a subtilase.

The control sequence may be an appropriate promoter sequence, a nucleic acid sequence that is recognized by a host cell for expression of the nucleic acid sequence. The promoter sequence contains transcriptional control sequences that mediate the expression of the subtilase. The promoter may be any nucleic acid sequence which shows transcriptional activity in the host cell of choice including mutant, truncated, and hybrid promoters, and may be obtained from genes encoding extracellular or intracellular subtilases either homologous or heterologous to the host cell.

Examples of suitable promoters for directing the transcription of the nucleic acid constructs of the present invention, especially in a bacterial host cell, are the promoters obtained from the *E. coli lac* operon, *Streptomyces coelicolor* agarase gene *(dagA), Bacillus subtilis* levansucrase gene *(sacB), Bacillus licheniformis* alpha-amylase gene (*amyL*), *Bacillus stearothermophilus* maltogenic amylase gene (*amyM*), *Bacillus amyloliquefaciens* alpha-amylase gene *(amyQ), Bacillus licheniformis* penicillinase gene (*penP*), *Bacillus subtilis xylA* and *xylB* genes, and prokaryotic beta-lactamase gene (Villa-Kamaroff *et al*., 1978, *Proceedings of the National Academy of Sciences USA* 75:

3727-3731), as well as the *tac* promoter (DeBoer *et al*., 1983, *Proceedings of the National Academy of Sciences USA* 80: 21-25). Further promoters are described in "Useful proteins from recombinant bacteria" in *Scientific American*, 1980, 242: 74-94; and in Sambrook *et al*., 1989, *supra.*

The control sequence may also be a suitable transcription terminator sequence, a sequence recognized by a host cell to terminate transcription. The terminator sequence is operably linked to the 3' terminus of the nucleic acid sequence encoding the subtilase. Any terminator which is functional in the host cell of choice may be used in the present invention.

The control sequence may also be a suitable leader sequence, a nontranslated region of an mRNA that is important for translation by the host cell. The leader sequence is operably linked to the 5' terminus of the nucleic acid sequence encoding the polypeptide. Any leader sequence that is functional in the host cell of choice may be used in the present invention.

The control sequence may also be a polyadenylation sequence, a sequence operably linked to the 3' terminus of the nucleic acid sequence and which, when transcribed, is recognized by the host cell as a signal to add polyadenosine residues to transcribed mRNA. Any polyadenylation sequence which is functional in the host cell of choice may be used in the present invention.

The control sequence may also be a signal peptide coding region that codes for an amino acid sequence linked to the amino terminus of a subtilase and directs the encoded subtilase into the cell's secretory pathway. The 5' end of the coding sequence of the nucleic acid sequence may inherently contain a signal peptide coding region naturally linked in translation reading frame with the segment of the coding region which encodes the secreted subtilase. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the subtilase. However, any signal peptide coding region which directs the expressed subtilase into the secretory pathway of a host cell of choice may be used in the present invention.

Effective signal peptide coding regions for bacterial host cells are the signal peptide coding regions obtained from the genes for *Bacillus* NCIB 11837 maltogenic amylase, *Bacillus stearothermophilus* alpha-amylase, *Bacillus licheniformis* subtilisin, *Bacillus licheniformis* beta-lactamase, *Bacillus stearothermophilus* neutral proteases (*nprT, nprS, nprM*), and *Bacillus subtilis prsA*. Further signal peptides are described by Simonen and Palva, 1993, *Microbiological Reviews* 57: 109-137.

The control sequence may also be a propeptide coding region that codes for an amino acid sequence positioned at the amino terminus of a subtilase. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (*aprE*), *Bacillus subtilis* neutral protease (*nprT*), *Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of a subtilase, the propeptide region is positioned next to the amino terminus of a subtilase and the signal peptide region is positioned next to the amino terminus of the propeptide region.

It may also be desirable to add regulatory sequences that allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used.

<u>Expression vectors/integration cassette</u>

A recombinant expression vector comprising a DNA construct of the invention may be any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome in part or in its entirety and replicated together with the chromosome(s) into which it has been integrated.

The vector may comprise an Integration cassette, which is designed to integrate into the host cell chromosome in one or more copies, where the cassette comprises the gene(s) of interest and optionally a selective marker such as an antibiotic resistance marker or a conditionally essential gene e.g. encoding galactose epimerase. Often an integration cassette is flanked or defined by polynucleotide sequence stretches of 50 to 500 bp that are identical or very homologous to host cell genomic sequences, and the integration into the host cell genome is achieved through homologous recombination between these stretches and the host cell genome.

The vector is preferably an expression vector in which the DNA sequence of the invention is operably linked to additional segments required for transcription of the DNA. In general, the expression vector is derived from plasmid or viral DNA, or may contain elements of both. The term, "operably linked" indicates that the segments are arranged so that they function in concert for their intended purposes, e.g. transcription initiates in a promoter and proceeds through the DNA sequence.

The promoter may be any DNA sequence which shows transcriptional activity in the host cell of choice and may be derived from genes encoding proteins either homologous or heterologous to the host cell.

Examples of suitable promoters for use in bacterial host cells include the promoter of the *Bacillus stearothermophilus* maltogenic amylase gene, the *Bacillus licheniformis* alpha-amylase gene, the *Bacillus amyloliquefaciens* alpha-amylase gene, the *Bacillus subtilis* alkaline protease gen, or the *Bacillus pumilus* xylosidase gene, or the phage Lambda $P_R$ or $P_L$ promoters or the E. coli lac, trp or tac promoters.

The DNA sequence of the invention may also, if necessary, be operably connected to a suitable terminator.

The recombinant vector of the invention may further comprise a DNA sequence enabling the vector to replicate in the host cell in question.

The vector may also comprise a selectable marker, e.g. a gene the product of which complements a defect in the host cell, or a gene encoding resistance to e.g. antibiotics like kanamycin, chloramphenicol, erythromycin, tetracycline, spectinomycine, or the like, or resistance to heavy metals or herbicides.

To direct an polypeptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the enzyme in the correct reading frame. Secretory signal sequences are commonly positioned 5' to the DNA sequence encoding the enzyme. The secretory signal sequence may be that normally associated with the enzyme or may be from a gene encoding another secreted protein.

The procedures used to ligate the DNA sequences coding for the present enzyme, the promoter and optionally the terminator and/or secretory signal sequence, respectively, or to assemble these sequences by suitable PCR amplification schemes, and to insert them into suitable vectors containing the information necessary for replication or integration, are well known to persons skilled in the art (cf., for instance, Sambrook et al., op.cit.).

## Host cell

The DNA sequence encoding the present enzyme introduced into the host cell may be either homologous or heterologous to the host in question. If homologous to the host cell, i.e. produced by the host cell in nature, it will typically be operably connected to another promoter sequence or, if applicable, another secretory signal sequence and/or terminator sequence than in its natural environment. The term "homologous" is intended to include a DNA sequence encoding an enzyme native to the host organism in question. The term "heterologous" is intended to include a DNA sequence not expressed by the host cell in nature. Thus, the DNA sequence may be from another organism, or it may be a synthetic sequence.

The host cell into which the DNA construct or the recombinant vector of the invention is introduced may be any cell which is capable of producing the present enzyme and includes bacteria, yeast, fungi and higher eukaryotic cells.

Examples of bacterial host cells which, on cultivation, are capable of producing the enzyme or complex of the invention are gram-positive bacteria such as strains of *Bacillus,* such as strains of *B. subtilis, B. licheniformis, B. lentus, B. brevis, B. stearothemtophilus, B. alkalophilus, B. amyloliquefaciens, B. coagulans, B. circulans, B. lautus, B. megatherium* or *B. thuringiensis,* or strains of *Streptomyces,* such as *S. lividans* or *S. murinus*, or gram-negative bacteria such as *Echerichia coli.* The transformation of the bacteria may be effected by protoplast transformation, electroporation, conjugation, or by using competent cells in a manner known per se (cf. Sambrook et al., supra).

When expressing the enzyme in bacteria such as *E. coli*, the enzyme may be retained in the cytoplasm, typically as insoluble granules (known as inclusion bodies), or may be directed to the periplasmic space by a bacterial secretion sequence. In the former case, the cells are lysed and the granules are recovered and denatured after which the enzyme is refolded by diluting the denaturing agent. In the latter case, the enzyme may be recovered from the periplasmic space by disrupting the cells, e.g. by sonication or osmotic shock, to release the contents of the periplasmic space and recovering the enzyme.

When expressing the enzyme in gram-positive bacteria such as Bacillus or Streptomyces strains, the enzyme may be retained in the cytoplasm, or may be directed to the extracellular medium by a bacterial secretion sequence. In the latter case, the enzyme may be recovered from the medium as described below.

Method of producing protease and/or protease inhibitor complex

The present invention provides a method of producing an isolated protease and/or protease inhibitor complex according to the invention, wherein a suitable host cell, which has been transformed with a DNA sequence encoding the protease and/or protease inhibitor complex, is cultured under conditions permitting the production of the complex, and the resulting complex or protease is recovered from the culture.
When an expression vector comprising a DNA sequence encoding the protein is transformed into a heterologous host cell it is possible to enable heterologous recombinant production of the complex of the invention.
Thereby it is possible to make a highly purified subtilase composition, characterized in being free from homologous impurities.
In this context homologous impurities mean any impurities (e.g. other polypeptides than the complex or protease of the invention) that originate from the homologous cell where the protein of the invention is originally obtained from.
The medium used to culture the transformed host cells may be any conventional medium suitable for growing the host cells in question. The expressed subtilase complex may conveniently be secreted into the culture medium and may be recovered from there by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

Use of a protease or complex of the invention

A complex of the invention may be used for a number of industrial applications, in particular within the detergent industry. Thus, the present invention also relates to a cleaning or detergent composition, preferably a laundry or dishwash composition comprising the complex of the invention.
In general, cleaning and detergent compositions are well described in the art and reference is made to WO 96/34946; WO 97/07202; WO 95/30011 for further description of suitable cleaning and detergent compositions.

Detergent compositions comprising the protease or complex of the invention

In general, cleaning and detergent compositions are well described in the art and reference is made to WO 96/34946; WO 97/07202; WO 95/30011 for further description of suitable cleaning and detergent compositions.

Detergent Compositions

The enzyme of the invention may be added to and thus become a component of a cleaning or detergent composition.
The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.
In a specific aspect, the invention provides a detergent additive comprising the protease or complex of the invention. The detergent additive as well as the detergent composition may comprise one or more other enzymes such as another protease, a lipase, a cutinase, an amylase, a carbohydrase, a cellulase, a pectinase, a mannanase, an arabinase, a galactanase, a xylanase, an oxidase, e.g., a laccase, and/or a peroxidase.
In general the properties of the chosen enzyme(s) should be compatible with the selected detergent, (i.e. pH-optimum, compatibility with other enzymatic and non-enzymatic ingredients, etc.), and the enzyme(s) should be present in effective amounts.
Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.
Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235, 252, 255, 259 and 274.
Preferred commercially available protease enzymes include Alcalase®, Savinase®, Primase®, Duralase®, Esperase®, and Kannase® (Novo Nordisk A/S), Maxatase®, Maxacal®, Maxapem®, Properase®, Purafect®, Purafect OxP®, FN2®, FN3®, and FN4® (Genencor Intemational Inc.).

**Detailed description of the Invention**

A method of screening a protease gene library for a gene encoding a protease of interest, the method comprising the steps of:

a) constructing a host cell comprising a first gene of the protease gene library and a second gene encoding a protease inhibitor;
b) cultivating the host cell, wherein the cell expresses the first and the second genes to produce a complex of a protease and the inhibitor,
c) dissociating the inhibitor from the complex; and
d) selecting the protease of interest and isolating the encoding gene.

A preferred host cell genus of the industrial enzyme manufacturers is *Bacillus,* especially cells of the species *Bacillus alkalophilus, Bacillus amyloliquefaciens, Bacillus brevis, Bacillus circulans, Bacillus coagulans, Bacillus lautus, Bacillus lentus, Bacillus licheniformis, Bacillus megaterium, Bacillus stearothermophilus, Bacillus subtilis,* and *Bacillus thuringiensis*.

Accordingly a preferred embodiment relates to a method of the first or second aspect, wherein the host cell is a prokaryotic cell, preferably of the *Bacillus* genus, more preferably of the species *B. licheniformis, B. clausii,* or *B. subtilis.*

Many ways are known in the art, by which the construction of a host cell comprising the two genes according to step a) of the first aspect can be done. The genes may be introduced into the cell on an extrachromosomal element, self-replicating or not, carrying antibiotic markers or not, expressed from regulated or constitutive promoters; the method of constructing the cell is of little importance in the context of the present invention, what is important is that essentially all extracellular protease is produced as part of a proteolytically inactive complex comprising a protease part and an inhibitor part.

However a preferred embodiment relates to a method of the first or second aspect, wherein the first and second genes are comprised in a genetic construct, preferably a plasmid, more preferably an integration cassette; preferably the first and second genes are expressed from separate promoters, and more preferably the separate promoters are identical.

Yet another preferred embodiment relates to a method of the first or second aspect, wherein the first and second genes are expressed as a polycistronic messenger from one or more promoter elements.

Still another preferred embodiment relates to a method of the first or second aspect, wherein the first and second genes are fused *in frame* to form a fusion polynucleotide encoding a fusion polypeptide.

The fusion polynucleotide of the previous embodiment comprises two genes fused *in frame* as described, and the two genes may even be separated by spacing nucleotides, as long as the genes remain *in frame* and together with the spacing sequence encodes a fusion polypeptide.

Consequently in a preferred embodiment of the first or second aspect the fusion polynucleotide further comprises a spacer of at least 6 basepairs between the two genes.

As described above, several classes of proteases are known in the art, especially the subtilases are well characterized. This invention particularly relates to subtilases of the I-S1 and I-S2 classes. A preferred embodiment of the invention relates to the method of the first aspect, wherein the protease gene library consists of genes encoding subtilases, preferably subtilases of the SI1 or SI2 group.

More specifically, a preferred embodiment relates to the method of the first aspect, wherein the protease gene library consists of genes encoding proteases derived from *Bacillus,* preferably subtilisin 309, subtilisin 168, subtilisin 147, subtilisin Novo, subtilisin Carlsberg, subtilisin BLAP, subtilisin PB92, subtilisin BPN or BPN', or variants thereof.

Another preferred embodiment relates to the method of the first aspect, wherein the protease gene library consists of shuffled genes resulting from shuffling homologous protease encoding genes.

Also a preferred embodiment relates to the method of the first aspect, wherein the protease gene library consists of shuffled genes resulting from shuffling heterologous protease encoding genes.

A more preferred embodiment relates to the method of the first aspect, wherein the protease gene library consists of genes encoding subtilisin 309 or variants thereof, preferably the variants comprise one or more of the modifications Y167A, R170S, and A194P.

Another preferred embodiment relates to the method of the first aspect, wherein the variants of subtilisin 309 comprise one or more of the modifications as follows:

M222S,
M222A+G195E,
*36D+N76D+N120D+G195E+K235L,
Y167A+R170S+A194P,
S87N+S101G+V104N,
S87N+M222S,
Y217L,
K27R+V104Y+N123S+T274A,
N76D+S103A+V104I, or
S101 G+S103A+V104I+G159D+ A232V+Q236H+Q245R+N248D+N252K.

We have previously described a number of CI-2A variants in WO 92/05239; WO 93/13125; WO 93/17086; and WO 93/20175, and a number of other protease inhibitors are described in the art.

A preferred embodiment relates to the method of the first aspect, wherein the second gene encodes a *Streptomyces* Subtilisin Inhibitor (SSI), an Eglin C inhibitor, a pumpkin trypsin inhibitor (CMTI), or a barley chymotrypsin inhibitor (CI); preferably the second gene encodes a barley chymotrypsin inhibitor; more preferably a CI-2A inhibitor (SEQ ID 1) or a variant thereof, and even more preferably a CI-2A inhibitor variant which has had an amino acid residue at one or more of the positions P6, P5, P4, P3, P2, P1, P'1, P'2, or P'3 substituted with another amino acid residue, still more preferably the variant of CI-2A comprises one or more of the following amino acid substitutions at the indicated position:

P6: Ala, Glu, Tyr, Pro or Lys
P5: Gly, Val, Leu, Glu, Ile or Pro
P4: Val, Pro, Trp, Ser, Glu, Gly, Lys or Arg
P3: Tyr, Glu, Ala, Arg, Pro, Ser, Lys, or Trp
P2: Ser, Lys, Arg, Pro, Glu, Val, Tyr, Trp, Ile, Gly or Ala
P1: Arg, Tyr, Trp, Glu, Val, Ser, Lys, Asp, Ile, Gly, or Ala
P'1: Gln, Ser, Thr, Ile, Lys, Asn, or Pro
P'2: Val, Glu, Arg, Pro, Gly or Trp
P'3: Glu, Gln, Asn, Val, Phe, Ile, Thr or Tyr;

and most preferably the variant of CI-2A comprises a proline at position P1 (M59P).

An optional part of the fusion polynucleotide sequence of the first aspect is a spacer or linker between the subtilase and the inhibitor encoding parts.

A preferred embodiment relates to the method of the first aspect, wherein the spacer encodes a peptide of a size of about 5 - 80 amino acids, preferably about 8 - 40 amino acids, and more preferably about 10 - 30 amino acids; more preferably the spacer encodes a peptide of a size of at least 15 amino acids.

A more preferred embodiment relates to the method of the first aspect, wherein the spacer encodes the amino acid sequence HAHAHSVSQEASVTR (SEQ ID 2).

It may be an advantage when assaying a library of proteases with varying catalytic activity to be able to directly compare the obtained results without having to correlate with enzyme concentrations afterwards. This can be achieved by first normalizing the amount of enzyme and then performing the activity assay.

Accordingly a preferred embodiment relates to the method of the first aspect, wherein the cultivation of the host cell in step b) is followed by an additional step of recovering essentially equimolar amounts of complex from each cell and using these recovered amounts in the subsequent steps; preferably the recovering is achieved by fusing a polyhistidine-tag to the protease and recovering this construct on a solid support, preferably a Ni-NTA solid support.

As the protease-inhibitor complex of the invention remains essentially catalytically inactive, it is necessary to dissociate the inhibitor part from the complex in order to assay the proteolytic activity of the protease part, before selecting a protease-encoding gene of interest. Various detergent solutions are known in the art to dissociate inhibitors from such a complex and essentially all surface-active compounds, naturally occurring or artificial, may be used for this purpose.

A preferred embodiment relates to the method of the first aspect, wherein the protease inhibitor is dissociated from the complex in step c) by a detergent solution, preferably by a solution comprising Linear Alkylbenzene Sulfonate (LAS).

The final step of the method of the invention involves a selection of the protease part of the complex. Such a selection is made based on a property of interest of the protease and that property may be assayed by any of the very large number of assays known in the art e.g. activity based assays, lability assays, stability assays, allergenicity assays and High Throughput Screens etc. Also various automated assay set-ups are envisioned.

A preferred embodiment relates to the method of the first aspect, wherein the protease of interest in step d) is selected on the basis of results from a microtiter plate based assay, preferably a microtiter based washing assay, more preferably an automated miniwash assay.

Another preferred embodiment relates to the method of the first aspect, wherein the protease of interest in step d) is selected on the basis of results from an assay that is based on active site titration using an enzyme inhibitor, measurement of fluorescence polarization using a fluorescenfly labelled enzyme inhibitor or measurement of labelled anti-enzyme antibodies.

As mentioned above, one of the properties that the present invention may improve is allergenicity, as the allergenicity of proteases have been correlated with the proteolytic activity. The inhibition of the activity of the protease or the protease-inhibitor complex may result in much lower allergenicity as determined by an assay. Non-limiting examples of suitable allergenicity assays are exemplified below.

Hence a preferred embodiment relates to the method of the first aspect, wherein the protease of interest in step d) is selected on the basis of higher proteolytic activity, reduced allergenicity, improved thermostability, or improved thermoactivity.

A preferred embodiment relates to the method of the first aspect, wherein the protease of interest in step d) is selected on the basis of reduced allergenicity, preferably the allergenicity of the protease-inhibitor complex is reduced at least 3 times when compared to the allergenicity of the parent protease, preferably at least 10 times reduced, more preferably at least 50 times, even more preferably at least 100 times, still more preferably at least 500 times, yet more preferably at least 1,000 times, more preferably at least 5,000 times, and most preferably at least 10,000 times.

The second aspect relates to a method of screening a protease inhibitor gene library for a gene encoding a protease inhibitor of interest, the method comprising the steps of:

a) constructing a host cell comprising a first gene encoding a protease and a second gene of the protease inhibitor library;
b) cultivating the host cell, wherein the cell expresses the first and the second genes to produce a complex of the protease and an inhibitor;
c) dissociating the inhibitor from the complex; and
d) selecting the inhibitor of interest and isolating the encoding gene.

The method provides the means to select an optimal protease inhibitor for any given protease, and the selection of an inhibitor from a library of inhibitors can be based on one or more properties of said inhibitor e.g. the constant of interaction, $K_I$, for the given protease, temperature and/or pH stability/lability, resistance to proteolytic degradation etc. Such properties can be assayed through one or more of the many assays known in the art, for instance in a non-limiting example the proteolytic activity of the pure protease part can be compared with the proteolytic activity of the complex under certain circumstances, where the complex ideally should be completely dissociated into its two parts. We do not envision any limitations as to what proteases or what protease inhibitors may be applied in the method of the invention.

A preferred embodiment relates to a method of the second aspect, wherein the first gene encodes a subtilase, preferably a subtilase of the SI1 or SI2 group.

Another preferred embodiment relates to a method of the second aspect, wherein the first gene encodes a protease derived from *Bacillus,* preferably subtilisin 309, subtilisin 168, subtilisin 147, subtilisin Novo, subtilisin Carlsberg, subtilisin BLAP, subtilisin PB92, subtilisin BPN or BPN', or a variant thereof.

Still another preferred embodiment relates to a method of the second aspect, wherein the protease inhibitor gene library consists of shuffled genes resulting from shuffling homologous protease inhibitor encoding genes.

One preferred embodiment relates to a method of the second aspect, wherein the protease inhibitor gene library consists of shuffled genes resulting from shuffling heterologous protease inhibitor encoding genes.

Yet a preferred embodiment relates to a method of the second aspect, wherein the protease inhibitor gene library consists of genes encoding *Streptomyces* Subtilisin Inhibitors (SSI), Eglin C inhibitors, pumpkin trypsin inhibitors (CMTI), or barley chymotrypsin inhibitors (CI).

One more preferred embodiment relates to a method of the second aspect, wherein the protease inhibitor gene library consists of genes encoding barley chymotrypsin inhibitors; preferably CI-2A inhibitors or variants thereof, and more preferably CI-2A inhibitor variants which have had an amino acid residue at one or more of the positions P6, P5, P4, P3, P2, P1, P'1, P'2, or P'3 substituted with another amino acid residue.

Final preferred embodiments relate to a protease of the third aspect, which is in a complex with a protease inhibitor, and a protease inhibitor of the fourth aspect, which is in a complex with a protease.

## EXAMPLES

Proteolytic activity

In the context of this invention proteolytic activity is expressed in Kilo Novo Protease Units (KNPU). The activity is determined relatively to an enzyme standard (Savinase®), and the determination is based on the digestion of a dimethyl casein (DMC) solution by the proteolytic enzyme at standard conditions, i.e. 50°C, pH 8.3, 9 min. reaction time, 3 min. measuring time. A folder AF 220/1 is available upon request to Novo Nordisk A/S, Denmark.

Determination of the constant of interaction, $K_i$, of an inhibitor

The constant of interaction, Ki, of an inhibitor for a subtilase can be determined in the following manner:

The concentration of an inhibitor preparation is estimated from the absorbance measured at 280nm using theoretically calculated extinction coefficients (Gill and von Hippel, 1989, *Anal Biochem.* 182:319-326) while the exact concentration of active inhibitor is determined using titration with a preparation of subtilisin 309 that has been active site titrated with N-trans-cinnamoyl imidazole (Schonbaum *et al.*, 1961, *J Biol Chem.* 236:2930-2935; Bender *et al.*, 1966, *J Am Chem Soc.* 88:5890-5913).

For the equilibrium between an enzyme and an inhibitor the following relationship exists:

$$[E] = ½\{[E_0] - [I_0] - K_i + \sqrt{([E_0] + [I_0] + [K_i])^2 - 4[E_0][I_0]}\}$$

meaning that $K_i$ can be calculated when [E] has been determined as a function of $[I_0]$. $[E_0]$ and $[I_0]$ are the initial concentrations of enzyme and inhibitor respectively, and [E] is the concentration of free enzyme at equilibrium.

Determination of the $K_i$ values for CI-2A and variants is carried out as follows. In a total volume of 1800µl, fixed amounts of subtilisin 309 is incubated in the absence of inhibitor or in the presence of varying amounts of inhibitor. At different time points 90µl incubation mixture is assayed for residual enzymatic activity through addition of 10µl of a chromogenic substrate in a Cobas Fara automated spectrophotometer. The absorbance at 410nm is measured every five seconds for 250 seconds. The reactions are carried out in 0.1M Tris-HCl, pH 8.6 @ 25°C and the final concentration of the chromogenic substrate is 1mM. Depending on the $K_i$ value of the inhibitor in question the $[E_0]$ used is between $2x10^{-10}$M (low $K_i$ values) and $1x10^{-7}$M (high $K_i$ values) while $[I_0]$ in general is varied from 25% to 250% of $[E_0]$. Typically ten different $[I_0]$ values are investigated. As chromogenic substrates Suc-Ala-Ala-Pro-Phe-pNA (inhibitors with low $K_i$ values) and Suc-Ala-Ala-Ala-pNA (inhibitors with high $K_i$ values) are used.

Ki values are calculated from plots of [E] versus $[I_0]$ using the non-Ilinear regression data analysis program Enzfitter (Leatherbarrow, 1987, Elsevier Science Publishers). The $K_i$ values determined are apparent as [E] turns out to be dependent on the concentration of the chromogenic substrate used to assay [E]. $K_i$(apparent) is related to $K_i$ through:

$$K_i(\text{apparent}) = K_i\{1+[S_0]/K_m\};$$

Where $[S_0]$ is the initial substrate concentration.

### Example 1

**Expression cassettes for simultaneous expression of the apr gene encoding subtilisin 309 or Savinase® from *Bacillus lentus* and CI-2A from barley**

The DNA material for the constructions below was isolated form the following sources:

The gene encoding the alkaline protease subtilisin 309 (Savinase®) from *B. lentus* NCIB 10309 was cloned and inserted in a derivative of pE194 (pPL2002)(Appl Envir Microbiol, 2000, 66(2):825-827).

The CI-2A chymotrypsin inhibitor encoding gene of barley and the plasmid carrying the gene translated through an alfa leader sequence were described in US patent No. 5,674,833 (1997). The CI-2A(M59P) chymotrypsin inhibitor was described in WO 92/05239 (Novo Nordisk).

The amyL promoter region was isolated from a derivative of *B.licheniformis* ATCC 9789.

The DNA segments were amplified and joined together mainly by the polymerase chain reaction (PCR) and the sequence overlap extension (SOE) techniques. The stepwise constructions involved a number of PCR primers with convenient flanking tails of either restrictions enzyme sites or overlapping DNA segments for the SOE fusions. In the end the DNA fusion product was inserted in an appropriate vector for inserting the genes in the chromosome of either *Bacillus subtilis* or *B.lentus* through recombination between homologous sequences in the host and the incoming plasmid derivative.

Four different DNA expression cassettes were made for evaluating the simultaneous production of Savinase® and CI-2A, schematics of the four constructs are represented in fig. 1 A-D.

Construct A was made as outlined herein, however another version "Construct A Pro" was also prepared (similarly to description below), wherein a single amino acid modification was introduced in the CI-2A inhibitor, the methionine in position 59 (or P1) was substituted with a proline (M59P) as previously described in WO 92/05239.

<u>The DNA constructs were made in a stepwise manner:</u>

The CI-2A gene was amplified from a derivative of pYACl2 by PCR:

a) For the construction of Expression cassette "A" (fig.1), the CI-2A gene was amplified by the primers pep72 (SEQ ID 4) and pep16 (SEQ ID 5). The N-terminal part was further extended by an additional PCR round with pep63 (SEQ ID 6) and pep16 as primers and the first PCR product as template. The primers pep72 (SEQ ID 4) and pep63 (SEQ ID 6) added the DNA codons for the 15 amino acid spacer region as well as an Mlu1 site in frame with the Mlu1 site of primer pep5 (SEQ ID 7) (below) for amplification of the C-terminal of the Savinase® gene. Pep16 (SEQ ID 5) added a Bgl2 site to the end of the CI-2A sequence.

b) For the "B" and "C" constructs (fig.1) the CI-2A gene was amplified by the primers pep36 (SEQ ID 8) and pep16 (SEQ ID 5). The pep36 (SEQ ID 8) primer has an overlapping tail to the pep39 (SEQ ID 9) primer for the amplification of the PamyL signal below.

c) For the "D" construct (fig. 1) the CI-2A gene was amplified by the primers pep35 (SEQ ID 10) and pep16 (SEQ ID 5). The pep35 (SEQ ID 10) primer added a BspH1 site in the ATG start region of the CI-2A gene for the later fusion to PamyL.

The PamyL segments were amplified with DNA isolated from a derivative of *B.licheniformis* ATCC 9789 as template:

d) For the "B" (fig.1) construct the PamyL region was amplified by the primers pep65 (SEQ ID 11) and pep39 (SEQ ID 9). The pep65 (SEQ ID 11) primer added an Mlu1 site upstream of the shine dalgamo (SD) of amyl for the right fusion to the amplified C-terminal of the Savinase® gene. Pep39 (SEQ ID 9) has an overlapping sequence matching the pep36 (SEQ ID 8) tail in the PCR product of step b) above.

e) In the "C" and "D" constructs (fig.1) the PamyL region was amplified by the primers 8805 (SEQ ID 12) and pep39 (SEQ ID 9). The 8805 (SEQ ID 12) primer added a BamH1 site upstream of the promoter of amyL for the insertion of the CI-2A behind the Savinase® gene in pPL2002. As indicated above the pep39 (SEQ ID 9) primer made an in frame fusion to the amyL signal possible by fusion to the pep36 (SEQ ID 8) tail in the PCR product of step b) above.

The Savinase® segments were amplified with the pPL2002 plasmid as template:

f) For the "A" and "B" constructs (fig.1) the C-terminal part of the Savinase® gene was amplified by the primers pep5 (SEQ ID 7) and 20231 (SEQ ID 13). The pep5 (SEQ ID 7) primer inserted an Mlu1 site in the C-terminal of the coding region for Savinase®.

g) For the "A" and "B" constructs (fig.1) the terminator region of Savinase® was amplified using primer 22121 (SEQ ID 14) and 20446 (SEQ ID 15). The primer 22121 (SEQ ID 14) inserted a BamH1 site in the Savinase® end of the terminator.

CI-2A fused to the Savinase® terminator

h) For the "A" construct (fig. 1) the CI-2A PCR fragment of step a) above was digested with Bgl2, and the Savinase® terminator PCR product of step g) was digested by BamH1. After ligation of the obtained fragments, the fused PCR product was amplified by the primers pep63 (SEQ ID 6) and 137393 (SEQ ID 16). The primer 137393 (SEQ ID 16) added a BamH1 site just next to the end of the Savinase® terminator.

i) For the "B" construct (fig. 1) the CI-2A PCR fragment of step b) was digested by Bgl2, and the Savinase® terminator PCR product of step g) was digested by BamH1. After ligation of the two fragments, the fused PCR product was amplified by the primers pep39 (SEQ ID 9) and 137393 (SEQ ID 16).

amyL CI-2A fusions:

j) For the "B" construct (fig.1) the amyL PCR fragment in of step d) and the CI-2A PCR fragment of step i) were joined together by SOE using the primers pep65 (SEQ ID 11) and 137393 (SEQ ID 16).

k) For the "C" construct (fig.1) the amyL PCR fragment of step e) and the CI-2A PCR fragment of step c) were joined together by SOE using the primers 8805 (SEQ ID 12) and pep16 (SEQ ID 5).

l) For the "D" construct (fig.1) the amyL PCR fragment of step e) was cut by BspH1 and the signal part of the PCR fragment was isolated and mixed with the CI-2A PCR fragment of step c) which was also cut by BspH1, and after ligation the product was amplified by PCR using the primers 8805 (SEQ ID 12) and pep16 (SEQ ID 5).

Operon fusions to the Savinase® gene:

m) For the "A" construct (fig.1) the Savinase® C-terminal PCR fragment of step f) was cut by Mlu1 and mixed with the CI-2A PCR fragment of step h) after digestion with Mlu1, and ligated. The product was amplified by the primers 20231 (SEQ ID 13) and 137393 (SEQ ID 16).

n) For the "B" construct (fig.1) the Savinase® C-terminal PCR fragment of step f) was cut by Mlu1 and mixed with the CI-2A PCR fragment of step j) after digestion with Mlu1, and ligated. The product was amplified by the primers 20231 (SEQ ID 13) and 137393 (SEQ ID 16).

Plasmid integration vectors: (derivatives of pPL2002 integration vector):

o) The final part of the "A" construct (fig.1) was the vector part of the pPL2002 plasmid which was digested by BstX1 and BamH1 and ligated to the large fragment (containing the CI-2A part) of the PCR fragment of step m) which was likewise digested by BstX1 and BamH1. The ligation was transformed in parallel with the pE194 plasmid to a *B.subtilis (apr, npr*) strain selecting for chloramphenicol resistance at 30°C. The right plasmid was identified by restriction and PCR analysis. The sequence of a PCR fragment containing the total C-terminal region of the Savinase®-CI-2A fusion confirmed the right fusion product and is shown in SEQ ID 17.

p) The final part of the "B" construct (fig.1) was the vector part of the pPL2002 plasmid which was digested by BstX1 and BamH1 and ligated to the large fragment (containing the CI-2A part) of the PCR fragment of step n) which was likewise digested by BstX1 and BamH1. The ligation was transformed in parallel with the pE194 plasmid to a *B.subtilis* (*apr, npr*) strain selecting for chloramphenicol resistance at 30°C. The right plasmid was identified by restriction and PCR analysis. The sequence of a PCR fragment containing the total C-terminal region of the Savinase®-CI-2A fusion confirmed the right transcriptional fusion product and is shown in SEQ ID 18.

q) The final part of the "C" construct (fig.1) was the pPL2002 plasmid digested by BamH1 and then ligated to the PCR fragment of step k) which was first digested by Bgl2 and BamH1. The ligation was transformed in parallel with the pE194 plasmid to a *B.subtilis* (*apr, npr*) strain selecting for chloramphinicol resistance at 30°C. The right plasmid was identified by restriction and PCR analysis. The sequence of a PCR product containing the PamyL sig-CI-2A fusion confirmed the right fusion product and is shown in SEQ ID 19.

r) The final part of the "D" construct (fig.1) was the pPL2002 plasmid digested by BamH1 and then ligated to the PCR fragment of step l) which was first digested by Bgl2 and BamH1. The ligation was transformed in parallel with the pE194 plasmid to a *B.subtilis* (apr, npr) strain selecting for chloramphinicol resistance at 30°C. The right plasmid was identified by restriction and PCR analysis. The sequence of a PCR product containing the PamyL ATG-CI-2A fusion confirmed the right fusion product and is shown in SEQ ID 20.

**Example 2**

**Production of CI-2A and the CI-2A - protease complex in *Bacillus subtilis***

The pPL2002 derivatives in Example 1 were inserted into the chromosome of a protease negative derivative of *B.subtilis* DN497 (WO91/09129). At first a small part of the Savinase® gene was inserted in the amyE gene to establish homology, secondly the pPL2002 derivatives containing constructs A, B, C or D of example 1 (fig.1) were inserted in the chromosome by selecting for chloramphenicol.

After 4 days of fermentation in shake flasks in complex growth media, the culture broths were analysed for protease and CI-2A content. The CI-2A content was identified through precipitation with antibody IgG raised in rabbits against a CI-2A protein that was isolated from a Yeast transformant known to produce the CI-2A inhibitor.

The protease activity was partly inhibited by the co expressed CI-2A protein, but could be detected by antibody IgG raised against Savinase®. The four strains comprising each of the four constructs all produced protein recognized by both Savinase®-IgG as well as CI-2A-IgG.

The CI-2A product of strains comprising constructs A and B was further characterized through SDS PAGE and Western blot analysis - The Western blot made with CI-2A antibodies identified the CI-2A product to be around 8 kDal (Mw for CI-2A = 9249 dalton) for both the A and B construct, meaning that the A construct (fig.1) must have been maturated resulting in the production of a non-covalently linked protease inhibitor complex.

Further characterisation of the Savinase®-spacer-CI-2A complex:

First a more productive expression cassette for production in *B.subtilis* of the A construct (fig1) was made. The promoter region PmPqPsav was replaced by a stronger promoter and after this replacement the new A construct was integrated along with the *cat* gene of pC194 into the *amyE* locus of DN497. This new strain PP916 was almost without detectable protease activity.

**Example 3**

**A Molecular analysis of the Savinase®-CI-2A complex after fermentation in B.subtilis**

After 5 days of shake flask fermentation in complex growth media with PP916 the culture broth was analysed for protease and CI-2A content.

The supernatant was separated through a packed column and the activity against Suc-AAPF-pNA in all fractions was measured. The protease test was performed with or without 0.5% linear alkyl benzene sulphonate (LAS) which is known to dissociate the CI-2A Savinase® complex into an active protease part and a free CI-2A molecule. The protease activity test result confirmed that the major part of the protease was found as the protease inactive protease-CI-2A complex, less than 5% of the normal protease activity could be detected when LAS was not added.

A 95% pure fraction containing the Savinase® CI-2A complex was further analysed by mass spectrometric methods. This molecular weight analysis demonstrated that the protease fraction consist of the Savinase® molecule with a 5 amino acid HisAlaHisAlaHis tail and that the CI-2A part is degraded to three almost identical molecules. In the degraded CI-2A mixture the N-terminal part of the wild type CI-2A molecule had been removed, and three nearly identical fragments were found: aa 11 to 83, aa 12 to 83, and 15 to 83.

Standard wash performance tests were carried out using six different commercial wash detergents to compare the activities of the Savinase® CI-2A complex and the Savinase® CI-2A(M59P) complex with the commercially available Savinase® enzyme under standard wash conditions.

The Savinase® CI-2A complex showed no significant performance under these normal washing conditions except a little in Detergent 4 (results not shown), which has a very high LAS content resulting in close to 0.5% final LAS concentration during the wash, a concentration which our results above indicate is high enough to dissociate the CI-2A inhibitor from the protease.

The wash test of the Savinase® CI-2A(M59P) complex however demonstrated that the enzyme activity of the this complex was indistinguishable from the pure Savinase® under normal washing conditions (table 1) indicating that the CI-2A(M59P) inhibitor variant dissociates completely from the protease, leaving the protease fully active as compared to the pure protease in the wash test.

| | Detergent 1 | Detergent 2 | Detergent 3 | Detergent 4 | Detergent 5 | Detergent 6 |
|---|---|---|---|---|---|---|
| Savinase-CI-2A(M59P) | 19,1 | 29,2 | 14,2 | 28,3 | 17,8 | 15,6 |
| Savinase | 18,6 | 29,1 | 13,7 | 28,7 | 18 | 15,2 |
| Blank | 10,2 | 22,1 | 9,5 | 17,8 | 10,3 | 12,5 |

| Wash assay | |
|---|---|
| Detergent dose | 3.0 g/l |
| PH | 10.5 |
| Wash time | 15 min. |
| Temperature | 15°C |
| Water hardness | 6°dH |
| Enzymes | Subtilisin 309 (Savinase®) & CI-2A fusions listed. |
| Enzyme conc. | 10 nM |
| Test system | 150 ml glass beakers with a stirring rod |

(continued)

| Wash assay | |
|---|---|
| Textile/volume | 5 textile pieces (Ø 2.5 cm) in 50 ml detergent |
| Test material | EMPA117 from Center for Testmaterials, Holland |

The detergents used in the assay were 6 different commercially available washing detergents, however a simple model formulation could also be used. pH is adjusted to 10.5 which is within the normal range for a powder detergent. Many compositions of detergents are publicly available and well known to those in the art a simple model detergent (No. 95) is as follows:

| | |
|---|---|
| 25% | STP ($Na_5P_3O_{10}$) |
| 25% | $Na_2SO_4$ |
| 10% | $Na_2CO_3$ |
| 20% | LAS (Nansa 80S) |
| 5.0% | Nonionic tenside (Dobanol 25-7) |
| 5.0% | $Na_2Si_2O_5$ |
| 0.5% | Carboxymethylcellulose (CMC) |
| 9.5% | Water |

Water hardness is adjusted by adding $CaCl_2$ and $MgCl_2$ ($Ca^{2+}$:$Mg^{2+}$ = 2:1) to deionized water (see also Surfactants in Consumer Products - Theory, Technology and Application, Springer Verlag 1986). pH of the detergent solution is adjusted to pH 10.5 by addition of HCl.

Measurement of reflectance or reemmision (R) on the test material is done at 460 nm using a Macbeth ColorEye 7000 photometer (Macbeth, Division of Kollmorgen Instruments Corporation, Germany). The measurements are done according to the manufacturers protocol.

This wash test can be set up to run in an automated robotic assay set-up using microtiter plates and miniature textile swatches labelled with test stains as described in this example.

**Example 4**

**Determination of Savinase® concentration by fluorescence polarization measurements**

The protease inbibitor part of the complex of the invention can be dissociated from the complex as described elsewhere herein, whereafter the following method can be used to measure the proteolytic activity of the remaining protease part in terms of active site titration with a labelled protease inhibitor.

The CI-2 protease inhibitor is labelled by standard means with a fluorescent probe. After growth as described above, the amount of a protease (Savinase®, Novo Nordisk, Denmark) variant in a given microtiter plate well may be measured directly in the wells by addition of fluorescence-labeled inhibitor which upon binding to the protease changes rotational speed. The rotational speed may be monitored by fluorescence polarization analysis or by other means which monitors diffusion (e.g. fluorescence correlation spectroscopy). Since the amount of Savinase® variant may vary significantly between the individual wells, the fluorescently labeled CI-2 inhibitor is added in two or three steps of defined amounts and the fluorescence polarisation is measured after each CI-2 addition. The determined concentration of Savinase® variant in the individual well may be used to adjust the input volume from this well into the activity assay, or can be used to correct the obtained activity data, in order to determine the specific activity of the Savinase® variant of that well.

**Example 5**

**His-tagging/purification of proteases to achieve equal protein input from differentially expressing clones**

The DNA sequence encoding the protease Savinase® (Novo Nordisk A/S, Denmark) is translationally fused to a sequence encoding a His6 tag and libraries of Savinase®-His6 variants are produced and introduced into *Bacillus.* After standard growth, a limited number of Savinase® enzymes of each variant (about 10% of what is secreted by Bacillus carrying the wildtype Savinase gene) are immobilized in the wells of Ni-NTA microtiter plates. The unbound fraction including cells and excess Savinase® is removed, the plate washed once or twice in a buffer containing 5-20 mM Imidazole. The His-tagged Savinase® variants are released from the solid support by the addition of 250 mM Imidazole, and aliquots of the supematants from each well are used as input in a wash performance assay as described in the previous examples.

**Example 6**

**Assays for reduced allergenicity**

When fusion polynucleotides have been constructed based on the methods described in this invention, it is desirable to confirm the antibody binding capacity of the resulting complexes, functionality, immunogenicity and/or allergenicity using a purified preparation. For that use, the complex can be expressed in larger scale, purified by conventional techniques, and the antibody binding and functionality should be examined in detail using dose-response curves and e.g. direct or competitive ELISA (C-ELISA).

The potentially reduced allergenicity (which is likely, but not necessarily true for a protein with low antibody binding) should be tested in *in vivo* or *in vitro* model systems: e.g. *in vitro* assays for immunogenicity such as assays based on cytokine expression profiles or other proliferation or differentiation responses of epithelial and other cells incl. B-cells and T-cells. Further, animal models for testing allergenicity should be set up to test a limited number of protein variants that show desired characteristics in vitro. Useful animal models include the guinea pig intratracheal model (GPIT) (Ritz, et al. Fund. Appl. Toxicol., 21, pp. 31-37, 1993), mouse subcutaneous (mouse-SC) (WO 98/30682, Novo Nordisk), the rat intratracheal (rat-IT) (WO 96/17929, Novo Nordisk), and the mouse intranasal (MINT) (Robinson et al., Fund. Appl. Toxicol. 34, pp. 15-24, 1996) models.

The immunogenicity of a complex or protease is measured in animal tests, wherein the animals are immunised with the protein and the immune response is measured. Specifically, it is of interest to determine the allergenicity by repeatedly exposing the animals to the protein by the intratracheal route and following the specific IgG and IgE titers. Alternatively, the mouse intranasal (MINT) test can be used to assess the allergenicity.

However, the present inventors have demonstrated that the performance in ELISA correlates closely to the immunogenic responses measured in animal tests. To obtain a useful reduction of the allergenicity of a protein, the IgE binding capacity of the protein variant must be reduced to at least below 75 %, preferably below 50 %, more preferably below 25 % of the IgE binding capacity of the parent protein as measured by the performance in IgE ELISA, given the value for the IgE binding capacity of the parent protein is set to 100 %.

Thus an initial assessment of the immunogenicity and/or allergenicity can be made by measuring the antibody binding capacity or antigenicity of the protein using appropriate antibodies. This approach has also been used in the literature (WO 99/47680).

**Methods**

Immunisation of Brown Norway rats:

Twenty intratracheal (IT) immunisations were performed weekly with 0,100 ml 0.9% (wt/vol) NaCl (control group), or 0,100 ml of a protein dilution (~0,1-1 mg/ml). Each group contained 10 rats. Blood samples (2 ml) were collected from the eye one week after every second immunisation. Serum was obtained by blood clothing and centrifugation and analysed as indicated below.

Immunisation of Balb/C mice:

Twenty subcutaneous (SC) immunisations were performed weekly with 0.05 ml 0.9% (wt/vol) NaCl (control group), or 0,050 ml of a protein dilution (~0,01-0,1 mg/ml). Each group contained 10 female Balb/C mice (about 20 grams) purchased from Bomholdtgaard, Ry, Denmark. Blood samples (0,100 ml) were collected from the eye one week after every second immunisation. Serum was obtained by blood clothing and centrifugation and analysed as indicated below.

ELISA Procedure for detecting serum levels of IgE and IgG:

Specific IgG1 and IgE levels were determined using the ELISA specific for mouse or rat IgG1 or IgE. Differences between data sets were analysed by using appropriate statistical methods.

Activation of CovaLink plates:

A fresh stock solution of cyanuric chloride in acetone (10 mg/ml) is diluted into PBS, while stirring, to a final concentration of 1 mg/ml and immediately aliquoted into CovaLink NH2 plates (100 microliter per well) and incubated for 5 minutes at room temperature. After three washes with PBS, the plates are dryed at 50°C for 30 minutes, sealed with sealing tape, and stored in plastic bags at room temperature for up to 3 weeks.

Mouse anti-Rat IgE was diluted 200x in PBS (5 microgram/ml). 100 microliter was added to each well. The plates were coated overnight at 4 °C.

Unspecific adsorption was blocked by incubating each well for 1 hour at room temperature with 200 microliter blocking buffer. The plates were washed 3x with 300 microliter washing buffer.

Unknown rat sera and a known rat IgE solution were diluted in dilution buffer: Typically 10x, 20x and 40x for the unknown sera, and ½ dilutions for the standard IgE starting from 1 μg/ml. 100 microliter was added to each well. Incubation was for 1 hour at room temperature.

Unbound material was removed by washing 3x with washing buffer. The anti-rat IgE (biotin) was diluted 2000x in dilution buffer. 100 microliter was added to each well. Incubation was for 1 hour at room temperature. Unbound material was removed by washing 3x with washing buffer.

Streptavidin was diluted 1000x in dilution buffer. 100 microliter was added to each well. Incubation was for 1 hour at room temperature. Unbound material was removed by washing 3x with 300 microliter washing buffer. OPD (0.6 mg/ml) and $H_2O_2$ (0.4 microliter /ml) were dissolved in citrate buffer. 100 microliter was added to each well. Incubation was for 30 minutes at room temperature. The reaction was stopped by addition of 100 microliter $H_2SO_4$. The plates were read at 492 nm with 620 nm as reference.

Similar determination of IgG can be performed using anti Rat-IgG and standard rat IgG reagents.

Similar determinations of IgG and IgE in mouse serum can be performed using the corresponding species-specific reagents.

Direct IgE assay:

To determine the IgE binding capacity of protein variants one can use an assay, essentially as described above, but using sequential addition of the following reagents:

1) Mouse anti-rat IgE antibodies coated in wells;
2) Known amounts of rat antiserum containing igE against the parent protein;
3) Dilution series of the protein variant in question (or parent protein as positive control);
4) Rabbit anti-parent antibodies
5) HRPO-labelled anti-rabbit Ig antibodies for detection using OPD as described.

The relative IgE binding capacity (end-point and/or affinity) of the protein variants relative to that of the parent protein are determined from the dilution-response curves. The IgE-posifive serum can be of other animals (including humans that inadvertently have been sensitized to the parent protein) provided that the species-specific anti-IgE capture antibodies are changed accordingly.

Competitive ELISA (GELISA):

C-ELISA was performed according to established procedures. In short, a 96 well ELISA plate was coated with the parent protein. After proper blocking and washing, the coated antigen was incubated with rabbit anti-enzyme polyclonal antiserum in the presence of various amounts of modified protein (the competitor). The residual amount of rabbit antiserum was detected by horseraddish peroxidase-labelled pig anti-rabbit immunoglobulin.

## Example 7

### HTS screening assay for antifungal activity

An antifungal agent can either inhibit the outgrowth of spores, vegetative cells, or both. To identify an antifungal agent inhibiting vegetative cells, 50 μl samples of a liquid culture of a tester strain, e.g. *Botrytis cinerae,* are distributed into each well of a microtiter plate; 50 μl of samples of sterile culture supematants from cultures of bacterial cells, e.g. *Bacillus,* to be screened for secretion of antifungal activities (*e.g.* variants of an antimicrobial protease), are then added to each well of the microtiter plate. Optionally, instead of sterilizing the culture supematants, a tissue culture insert (e.g., Nunc TC Insert) may be inserted, to prevent contact between the secreting cells and cells of the tester strain. The insert may contain a membrane non-permeable to proteins or other macromolecular components, which allows the passage of for example small antimicrobial peptides. The microtiter plate is incubated at 30°C for 2-5 days whereafter antifungal activity is analysed by optical density measurements in each well at a suitable wavelength. Low optical density indicates the presence of an antifungal activity in the well.

## Claims

1. A method of screening a protease gene library for a gene encoding a protease of interest, the method comprising the steps of:

    a) constructing a host cell comprising a first gene of the protease gene library and a second gene encoding a protease inhibitor;
    b) cultivating the host cell, wherein the cell expresses the first and the second genes to produce a complex of a protease and the inhibitor;
    c) dissociating the inhibitor from the complex; and
    d) selecting the protease of interest and isolating the encoding gene.

2. The method of claim 1, wherein the host cell is a prokaryotic cell, preferably of the *Bacillus* genus, more preferably of the species *B. licheniformis, B. clausii,* or *B. subtilis.*

3. The method of claim 1 or 2, wherein the first and second genes are comprised in a genetic construct, preferably a plasmid, more preferably an integration cassette.

**4.** The method of claim 3, wherein the first and second genes are expressed from separate promoters.

**5.** The method of claim 4, wherein the separate promoters are identical.

**6.** The method of claim 3, wherein the first and second genes are expressed as a polycistronic messenger from one or more promoter elements.

**7.** The method of claim 3, wherein the first and second genes are fused *in frame* to form a fusion polynucleotide encoding a fusion polypeptide.

**8.** The method of claim 7, wherein the fusion polynucleotide further comprises a spacer of at least 6 basepairs between the two genes.

**9.** The method of any of claims 1 - 8, wherein the protease gene library consists of genes encoding subtilases, preferably subtilases of the SI1 or SI2 group.

**10.** The method of any of claims 1 - 8, wherein the protease gene library consists of genes encoding proteases derived from *Bacillus,* preferably subtilisin 309, subtilisin 168, subtilisin 147, subtilisin Novo, subtilisin Carlsberg, subtilisin BLAP, subtilisin PB92, subtilisin BPN or BPN', or variants thereof.

**11.** The method of any of claims 1 - 8, wherein the protease gene library consists of shuffled genes resulting from shuffling homologous protease encoding genes.

**12.** The method of any of claims 1 - 8, wherein the protease gene library consists of shuffled genes resulting from shuffling heterologous protease encoding genes.

**13.** The method of any of claims 1 - 12, wherein the second gene encodes *a Streptomyces* Subtilisin Inhibitor (SSI), an Eglin C inhibitor, a pumpkin trypsin inhibitor (CMTI), *or a* barley chymotrypsin inhibitor (CI).

**14.** The method of any of claims 1 - 12, wherein the second gene encodes a barley chymotrypsin inhibitor; preferably a CI-2A inhibitor (SEQ ID 1) or a variant thereof, and more preferably a CI-2A inhibitor variant which has had an amino acid residue at one or more of the positions P6, P5, P4, P3, P2, P1, P'1, P'2, or P'3, corresponding to amino acid residues 54-62 of SEQ ID, substituted with another amino acid residue.

**15.** The method of claim 14, wherein the variant of CI-2A comprises one or more of the following amino acid substitutions at the indicated position:

P6: Ala, Glu, Tyr, Pro or Lys
P5: Gly, Val, Leu, Glu, Ile or Pro
P4: Val, Pro, Trp, Ser, Glu, Gly, Lys or Arg
P3: Tyr, Glu, Ala, Arg, Pro, Ser, Lys, or Trp
P2: Ser, Lys, Arg, Pro, Glu, Val, Tyr, Trp, Ile, Gly or Ala
P1: Arg, Tyr, Trp, Glu, Val, Ser, Lys, Asp, lie, Gly, or Ala
P'1: Gln, Ser, Thr, Ile, Lys, Asn, or Pro
P'2: Val, Glu, Arg, Pro, Gly or Trp
P'3: Glu, Gln, Asn, Val, Phe, Ile, Thr or Tyr.

**16.** The method of claim 14, wherein the variant of CI-2A comprises a proline at position P1 (M59P).

**17.** The method of any of claims 8 - 16, wherein the spacer encodes the amino acid sequence HAHAHSVSQEASVTR (SEQ ID 2).

**18.** The method of any of claims 1 - 17, wherein the cultivation of the host cell in step b) is followed by an additional step of recovering essentially equimolar amounts of complex from each cell and using these recovered amounts in the subsequent steps.

**19.** The method of claim 18, wherein the recovering is achieved by fusing a polyhistidine-tag to the protease and recovering this construct on a solid support, preferably a Ni-NTA solid support.

**20.** The method of any of claims 1 - 19, wherein the protease inhibitor is dissociated from the complex in step c) by a detergent solution, preferably by a solution comprising Linear Alkylbenzene Sulfonate (LAS).

**21.** The method of any of claims 1 - 20, wherein the protease of interest in step d) is selected on the basis of results from a microtiter plate based assay, preferably a microtiter based washing assay, more preferably an automated miniwash assay.

**22.** The method of any of claims 1 - 20, wherein the protease of interest in step d) is selected on the basis of results from an assay that is based on active site titration using an enzyme inhibitor, measurement of fluorescence polarization using a fluorescently labelled enzyme inhibitor or measurement of labelled anti-enzyme antibodies.

**23.** The method of any of claims 1 - 22, wherein the protease of interest in step d) is selected on the basis of higher proteolytic activity, reduced allergenicity, improved thermostability, or improved thermoactivity.

**Patentansprüche**

**1.** Verfahren zum Screening einer Protease-Genbibliothek nach einem Gen, das eine interessierende Protease kodiert, wobei das Verfahren die Schritte umfasst:

(a) Konstruieren einer Wirtszelle, welche ein erstes Gen der Protease-Genbiliothek und ein zweites Gen, welches einen Protease-Inhibitor kodiert, umfasst;
(b) Kultivieren der Wirtszelle, wobei die Zelle das erste und das zweite Gen exprimiert, um einen Komplex aus einer Protease und dem Inhibitor herzustellen;
(c) Abtrennen des Inhibitors von dem Komplex; und
(d) Selektieren der interessierenden Protease und Isolieren des kodierenden Gens.

**2.** Verfahren nach Anspruch 1, in welchem die Wirtszelle eine prokaryotische Zelle, bevorzugt eine der Gattung *Bacillus,* stärker bevorzugt eine der Arten *B. licheniformis, B. clausii* oder *B. subtilis,* ist.

**3.** Verfahren nach Anspruch 1 oder 2, in welchem das erste und das zweite Gen von einem genetischen Konstrukt, bevorzugt einer Integrationskassette, umfasst sind.

**4.** Verfahren nach Anspruch 3, in welchem das erste und das zweite Gen von separaten Promotoren exprimiert werden.

**5.** Verfahren nach Anspruch 4, in welchem die separaten Promotoren identisch sind.

**6.** Verfahren nach Anspruch 3, in welchem das erste und das zweite Gen als polycistronischer Bote von einem oder mehreren Promotorelementen exprimiert werden.

**7.** Verfahren nach Anspruch 3, in welchem das erste und das zweite G*en im Rahmen* ("*in frame"*) fusioniert sind, um ein Fusionspolynukleotid zu bilden, das ein Fusionspolypeptid kodiert.

**8.** Verfahren nach Anspruch 7, in welchem das Fusionspolypeptid weiterhin einen Abstandshalter von mindestens 6 Basenpaaren zwischen den zwei Genen umfasst.

**9.** Verfahren nach einem beliebigen der Ansprüche 1 bis 8, in welchem die Protease-Genbibliothek aus Genen besteht, die Subtilasen kodieren, bevorzugt Subtilasen der SI1- oder SI2-Gruppe.

**10.** Verfahren nach einem beliebigen der Ansprüche 1 bis 8, in welchem die Protease-Genbibliothek aus Genen besteht, welche Proteasen kodieren, die von *Bacillus* stammen, bevorzugt Subtilisin 309, Subtilisin 168, Subtilisin 147, Subtilisin Novo, Subtilisin Carlsberg, Subtilisin BLAP, Subtilisin PB92, Subtilisin BPN oder BPN', oder Varianten hiervon.

**11.** Verfahren nach einem beliebigen der Ansprüche 1 bis 8, in welchem die Protease-Genbibliothek aus gemischten Genen besteht, die aus dem Mischen homologer Proteasekodierender Gene resultiert.

**12.** Verfahren nach einem beliebigen der Ansprüche 1 bis 8, in welchem die Protease-Genbibliothek aus gemischten Genen besteht, die aus dem Mischen heterologer Proteasekodierender Gene resultiert.

**13.** Verfahren nach einem beliebigen der Ansprüche 1 bis 12, in welchem das zweite Gen einen *Streptomyces* Subtilisin-Inhibitor (SSI), einen Eglin C-Inhibitor, einen Pumpkin Trypsin-Inhibitor (CMTI) oder einen Gersten ("barley") Chymotrypsin-Inhibitor (CI) kodiert.

**14.** Verfahren nach einem beliebigen der Ansprüche 1 bis 12, in welchem das zweite Gen einen Gersten Cymotrypsin-Inhibitor kodiert; bevorzugt einen CI-2A-Inhibitor (SEQ ID

1) oder eine Variante hiervon, und stärker bevorzugt eine CI-2A-Inhibitorvariante, die einen Aminosäurerest an einer oder mehreren der Positionen P6, P5, P4, P3, P2, P1, P'1, P'2 oder P'3, entsprechend den Aminosäureresten 54 bis 62 aus SEQ ID NR 1, besessen hatte, der durch einen anderen Aminosäurerest substituiert ist.

**15.** Verfahren nach Anspruch 14, in welchem die Variante von CI-2A eine oder mehrere der folgenden Aminosäuresubstitutionen an der angezeigten Position umfasst:

P6: Ala, Glu, Tyr, Pro oder Lys
P5: Gly, Val, Leu, Glu, Ile oder Pro
P4: Val, Pro, Trp, Ser, Glu, Gly, Lys oder Arg
P3: Tyr, Glu, Ala, Arg, Pro, Ser, Lys oder Trp
P2: Ser, Lys, Arg, Pro, Glu, Val, Tyr, Trp, Ile, Gly oder Ala
P1: Arg, Tyr, Trp, Glu, Val, Ser, Lys, Asp, Ile, Gly oder Ala
P'1: Gln, Ser, Thr, Ile, Lys, Asn oder Pro
P'2: Val, Glu, Arg, Pro, Gly oder Trp
P'3: Glu, Gln, Asn, Val, Phe, Ile, Thr oder Tyr.

**16.** Verfahren nach Anspruch 14, in welchem die Variante von CI-2A ein Prolin an der Position P1 (M59P) umfasst.

**17.** Verfahren nach einem beliebigen der Ansprüche 8 bis 16, in welchem der Abstandshalter die Aminosäuresequenz HAHAHSVSQEASVTR (SEQ ID 2) kodiert.

**18.** Verfahren nach einem beliebigen der Ansprüche 1 bis 17, in welchem auf die Kultivierung der Wirtszelle aus Schritt (b) ein zusätzlicher Rückgewinnungsschritt von im wesentlichen äquimolaren Mengen des Komplexes aus jeder Zelle und Verwenden dieser rückgewonnenen Mengen in den nachfolgenden Schritten folgt.

**19.** Verfahren nach Anspruch 18, in welchem die Rückgewinnung durch Fusionieren von einem Polyhistidin-Tag an die Protease und Rückgewinnung dieses Konstruktes über einen festen Träger, bevorzugt einen Ni-NTA festen Träger, erreicht wird.

**20.** Verfahren nach einem beliebigen der Ansprüche 1 bis 19, in welchem der Protease-Inhibitor von dem Komplex in Schritt (c) durch eine Detergenslösung, bevorzugt durch eine Lösung, die lineares Alkylbenzolsulfonat (LAS) umfasst, abgetrennt wird.

**21.** Verfahren nach einem beliebigen der Ansprüche 1 bis 20, in welchem die Protease von Interesse in Schritt (d) auf der Basis der Ergebnisse eines Mikrotiterplatten-basierten Assays, bevorzugt eines Mikrotiter-basierten Wasch-Assays, stärker bevorzugt eines automatisierten Miniwasch-Assays, ausgewählt wird.

**22.** Verfahren nach einem beliebigen der Ansprüche 1 bis 20, in welchem die Protease von Interesse in Schritt (d) auf der Basis der Ergebnisse eines Assays ausgewählt wird, das auf der Titration der aktiven Stelle unter Verwenden eines Enzym-Inhibitors, der Messung von Fluoreszenzpolarisation unter Verwenden eines Fluoreszenz-markierten Enzym-Inhibitors oder der Messung von markierten Anti-Enzym-Antikörpern basiert.

**23.** Verfahren nach einem beliebigen der Ansprüche 1 bis 22, in welchem die Protease von Interesse in Schritt (d) auf der Basis von höherer proteolytischer Aktivität, reduzierter Allergenität, verbesserter Thermostabilität oder verbesserter Thermoaktivität ausgewählt wird.

## Revendications

**1.** Procédé de criblage d'une banque de gènes de protéases pour un gène codant pour une protéase d'intérêt, le procédé comprenant les étapes de :

a) construction d'une cellule hôte comprenant un premier gène de la banque de gènes de protéases et un deuxième gène codant pour un inhibiteur de la protéase ;

b) culture de la cellule hôte, dans laquelle la cellule exprime le premier et le deuxième gènes pour produire un complexe d'une protéase et de l'inhibiteur ;

c) dissociation de l'inhibiteur du complexe ; et

d) sélection de la protéase d'intérêt et d'isolation du gène codant.

**2.** Procédé selon la revendication 1, dans lequel la cellule hôte est une cellule de procaryote, préférablement du genre *Bacillus,* plus préférablement de l'espèce *B. licheniformis, B. clausii*, ou *B. subtilis.*

**3.** Procédé selon la revendication 1 ou 2, dans lequel le premier et le deuxième gènes sont compris dans une construction génétique, préférablement un plasmide, plus préférablement une cassette d'intégration.

**4.** Procédé selon la revendication 3, dans lequel le premier et le deuxième gènes sont exprimés à partir de promoteurs séparés.

**5.** Procédé selon la revendication 4, dans lequel les promoteurs séparés sont identiques.

**6.** Procédé selon la revendication 3, dans lequel le premier et le deuxième gènes sont exprimés en tant que messager polycistronique à partir d'un ou plusieurs élément(s) de promoteur.

**7.** Procédé selon la revendication 3, dans lequel le premier et le deuxième gènes sont en phase de lecture pour former un polynucléotide de fusion codant pour un polypeptide de fusion.

**8.** Procédé selon la revendication 7, dans lequel le polynucléotide de fusion comprend en outre un espaceur d'au moins 6 paires de bases entre les deux gènes.

**9.** Procédé selon l'une quelconque des revendications 1 à 8, dans lequel la banque de gènes de protéases consiste en des gènes codant pour des subtilases, préférablement des subtilases du groupe SI1 ou SI2.

**10.** Procédé selon l'une quelconque des revendications 1 à 8, dans lequel la banque de gènes de protéases consiste en des gènes codant pour des protéases dérivées de *Bacillus,* de préférence la subtilisine 309, la subtilisine 168, la subtilisine 147, la subtilisine Novo, la subtilisine Carlsberg, la subtilisine BLAP, la subtilisine PB92, la subtilisine BPN ou BPN', ou des variants de celles-ci.

**11.** Procédé selon l'une quelconque des revendications 1 à 8, dans lequel la banque de gènes de protéases consiste en des gènes réarrangés résultant du réarrangement de gènes codant pour des protéases homologues.

**12.** Procédé selon l'une quelconque des revendications 1 à 8, dans lequel la banque de gènes de protéases consiste en des gènes réarrangés résultant du réarrangement de gènes codant pour des protéases hétérologues.

**13.** Procédé selon l'une quelconque des revendications 1 à 12, dans lequel le deuxième gène code pour un Inhibiteur de la Subtilisine de *Streptomyces* (SSI ; *Streptomyces* Subtilisin Inhibitor), un inhibiteur Eglin C, un inhibiteur de la trypsine de la citrouille (CMTI), ou un inhibiteur de la chymotrypsine de l'orge (CI).

**14.** Procédé selon l'une quelconque des revendications 1 à 12, dans lequel le deuxième gène code pour un inhibiteur de la chymotrypsine de l'orge ; préférablement un inhibiteur CI-2A (SEQ ID NO : 1) ou un variant de celui-ci, et plus préférablement un variant de l'inhibiteur CI-2A qui a eu un résidu d'acide aminé sur une ou plusieurs des positions P6, P5, P4, P3, P2, P1, P'1, P'2, ou P'3, correspondant aux résidus d'acides aminés 54 à 62 de SEQ ID NO : 1, substitué par un autre résidu d'acide aminé.

**15.** Procédé selon la revendication 14, dans lequel le variant de CI-2A comprend une ou plusieurs des substitutions d'acides aminés suivantes à la position indiquée :

P6 : Ala, Glu, Tyr, Pro ou Lys
P5 : Gly, Val, Leu, Glu, Ile ou Pro
P4 : Val, Pro, Trp, Ser, Glu, Gly, Lys ou Arg
P3 : Tyr, Glu, Ala, Arg, Pro, Ser, Lys, ou Trp
P2 : Ser, Lys, Arg, Pro, Glu, Val, Tyr, Trp, Ile, Gly ou Ala
P1 : Arg, Tyr, Trp, Glu, Val, Ser, Lys, Asp, Ile, Gly, ou Ala
P'1 : Gln, Ser, Thr, Ile, Lys, Asn, ou Pro
P'2 : Val, Glu, Arg, Pro, Gly ou Trp
P'3 : Glu, Gln, Asn, Val, Phe, Ile, Thr ou Tyr.

**16.** Procédé selon la revendication 14, dans lequel le variant de CI-2A comprend une proline en position P1 (M59P).

**17.** Procédé selon l'une quelconque des revendications 8 à 16, dans lequel l'espaceur code pour la séquence d'acides aminés HAHAHSVSQEASVTR (SEQ ID NO : 2).

**18.** Procédé selon l'une quelconque des revendications 1 à 17, dans lequel la culture de la cellule hôte à l'étape b) est suivie d'une étape additionnelle de récupération de quantités sensiblement équimolaires de complexe à partir de chaque cellule et d'utilisation de ces quantités récupérées dans les étapes subséquentes.

**19.** Procédé selon la revendication 18, dans lequel la récupération est réalisée en fusionnant un tag polyhistidine à la protéase et en récupérant cette construction sur un support solide, préférablement un support solide Ni-NTA.

**20.** Procédé selon l'une quelconque des revendications 1 à 19, dans lequel l'inhibiteur de la protéase est dissocié du complexe à l'étape c) par une solution détergente, préférablement par une solution comprenant du sulfonate d'alkylbenzène linéaire (LAS ; Linear Alkyl-benzene Sulfonate).

**21.** Procédé selon l'une quelconque des revendications 1 à 20, dans lequel la protéase d'intérêt à l'étape d) est sélectionnée sur la base des résultats d'un test sur plaques de microtitration, préférablement un test de lavage pour microtitration, plus préférablement un test de micro-lavage automatisé.

**22.** Procédé selon l'une quelconque des revendications 1 à 20, dans lequel la protéase d'intérêt à l'étape d) est sélectionnée sur la base des résultats d'un test qui est basé sur la titration de sites actifs en utilisant un inhibiteur d'enzyme, la mesure de la polarisation de fluorescence en utilisant un inhibiteur d'enzyme marqué de façon fluorescente ou la mesure d'anticorps anti-enzymes marqués.

**23.** Procédé selon l'une quelconque des revendications 1 à 22, dans lequel la protéase d'intérêt à l'étape d) est sélectionnée sur la base d'une activité protéolytique supérieure, d'une allergénicité réduite, d'une thermostabilité améliorée, ou d'une thermoactivité améliorée.

SEQUENCE LISTING

<110> Novo Nordisk A/S

<120> Method for screening highly active proteases and inhibitors

<130> 10080.000-DK

<140>
<141>

<160> 20

<170> PatentIn Ver. 2.1

<210> 1
<211> 83
<212> PRT
<213> Hordeum sp.

<220>
<223> barley chymotrypsin inhibitor CI-2A

<400> 1

```
Ser Ser Val Glu Lys Lys Pro Glu Gly Val Asn Thr Gly Ala Gly Asp
 1               5                  10                  15

Arg His Asn Leu Lys Thr Glu Trp Pro Glu Leu Val Gly Lys Ser Val
            20                  25                  30

Glu Glu Ala Lys Lys Val Ile Leu Gln Asp Lys Pro Glu Ala Gln Ile
        35                  40                  45

Ile Val Leu Pro Val Gly Thr Ile Val Thr Met Glu Tyr Arg Ile Asp
    50                  55                  60

Arg Val Arg Leu Phe Val Asp Lys Leu Asp Asn Ile Ala Gln Val Pro
65                  70                  75                  80

Arg Val Gly
```

<210> 2
<211> 15
<212> PRT
<213> Artificial sequence

<220>
<223> Description of Artificial sequence: Peptide linker/spacer

<400> 2

His Ala His Ala His Ser Val Ser Gln Glu Ala Ser Val Thr Arg
1 5 10 15

<210> 3
<211> 368
<212> PRT
<213> Artificial Sequence

<220>
<223> Description of Artificial sequence: Mature secreted fusion protein of subtilisin 309-linker-CI-2A(M59P).

<400> 3

```
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg His Ala His
            260                 265                 270

Ala His Ser Val Ser Gln Glu Ala Ser Val Thr Arg Met Ser Ser Val
        275                 280                 285

Glu Lys Lys Pro Glu Gly Val Asn Thr Gly Ala Gly Asp Arg His Asn
    290                 295                 300

Leu Lys Thr Glu Trp Pro Glu Leu Val Gly Lys Ser Val Glu Glu Ala
305                 310                 315                 320

Lys Lys Val Ile Leu Gln Asp Lys Pro Glu Ala Gln Ile Ile Val Leu
                325                 330                 335

Pro Val Gly Thr Ile Val Thr Pro Glu Tyr Arg Ile Asp Arg Val Arg
            340                 345                 350

Leu Phe Val Asp Lys Leu Asp Asn Ile Ala Gln Val Pro Arg Val Gly
```

355 360 365

<210> 4
<211> 52
<212> DNA
<213> Artificial sequence

<220>
<223> Description of Artificial sequence: Primer pep72

<400> 4

tcagtctcac aggaagcgag cgtaacaagg atgagttcag tggagaagaa gc 52

<210> 5
<211> 26
<212> DNA
<213> Artificial sequence

<220>
<223> Description of Artificial sequence: Primer pep16

<400> 5

ggaagatcta gccgaccctg gggacc 26

<210> 6
<211> 45
<212> DNA
<213> Artificial Sequence

<220>
<223> Description of Artificial sequence: Primer pep63

<400> 6

gcggcaacgc gtcatgctca cgcacattca gtctcacagg aagcg 45

<210> 7
<211> 23
<212> DNA
<213> Artificial Sequence

<220>
<223> Description of Artificial sequence: Primer pep5

<400> 7

attgattaac gcgttgccgc ttc 23

<210> 8
<211> 39
<212> DNA
<213> Artificial Sequence

<220>
<223> Description of Artificial sequence: Primer pep36

<400> 8

ctgcagcagc ggcggcaagt tcagtggaga agaagccgg 39

<210> 9
<211> 19
<212> DNA
<213> Artificial Sequence

<220>
<223> Description of Artificial sequence: Primer pep39

<400> 9

ttgccgccgg ctgctgcag 19

<210> 10
<211> 39
<212> DNA
<213> Artificial sequence

<220>
<223> Description of Artificial sequence: Primer pep35

<400> 10

gaaaggggag gagaatcatg agttcagtgg agaagaagc 39

<210> 11
<211> 34
<212> DNA
<213> Artificial Sequence

<220>
<223> Description of Artificial sequence: Primer pep65

<400> 11

gcggcaacgc gttaaaaatt cggaatattt atac 34

<210> 12
<211> 34
<212> DNA
<213> Artificial Sequence

<220> .
<223> Description of Artificial sequence: Primer 8805

<400> 12

cgggatccgt cgacggccgc agatgctgct gaag 34

<210> 13
<211> 21
<212> DNA
<213> Artificial Sequence

<220>
<223> Description of Artificial sequence: Primer 20231

<400> 13

gcccagaaga tgtggacgcg c 21

<210> 14
<211> 40
<212> DNA
<213> Artificial sequence

<220>
<223> Description of Artificial sequence: Primer 22121

<400> 14

gtcggagctc gtgcacgcgg atccagaagc ggcaacacgc 40

<210> 15
<211> 24
<212> DNA
<213> Artificial sequence

<220>
<223> Description of Artificial sequence: Primer 20446

<400> 15

aagctgatca gccctttacg ctcg 24

<210> 16
<211> 32
<212> DNA
<213> Artificial Sequence

<220>
<223> Description of Artificial sequence: Primer 137393

<400> 16

tttggatcca tacacaaaaa aacgctgtgc cc 32

<210> 17
<211> 2166
<212> DNA
<213> Artificial Sequence

<220>
<223> Description of Artificial sequence: construct A

<400> 17

gaattccggc ccaacgatgg ctgatttccg ggttgacggc cggcggaacc aaggggtgat 60
cggtcggcgg aaatgaaggc ctgcggcgag tgcgggcctt ctgttttgag gattataatc 120
agagtatatt gaaagtttcg cgatcttttc gtataattgt tttaggcata gtgcaatcga 180
ttgtttgaga aaagaagaag accataaaaa taccttgtct gtcatcagac agggtatttt 240
ttatgctgtc cagactgtcc gctgtgtaaa aataaggaat aaagggggggt tgttattatt 300
ttactgatat gtaaaatata atttgtataa gaaaatgaga gggagaggaa acatgattca 360
aaaacgaaag cggacagttt cgttcagact tgtgcttatg tgcacgctgt tatttgtcag 420
tttgccgatt acaaaaacat cagccgtaaa tggcacgctg atgcagtatt ttgaatggta 480
tacgccgaac gacggccagc attggaaacg attgcagaat gatgcggaac atttatcgga 540
ttaacttaac gttaatattt gtttcccaat aggcaaatct ttctaacttt gatacgttta 600
aactaccagc ttggacaagt tggtataaaa atgaggaggg aaaccgaatg aagaaaccgt 660
tggggaaaat tgtcgcaagc accgcactac tcatttctgt tgcttttagt tcatcgatcg 720
catcggctgc tgaagaagca aaagaaaaat atttaattgg ctttaatgag caggaagctg 780
tcagtgagtt tgtagaacaa gtagaggcaa atgacgaggt cgccattctc tctgaggaag 840
aggaagtcga aattgaattg cttcatgaat ttgaaacgat tcctgtttta tccgttgagt 900
taagcccaga agatgtggac gcgcttgaac tcgatccagc gatttcttat attgaagagg 960
atgcagaagt aacgacaatg gcgcaatcag tgccatgggg aattagccgt gtgcaagccc 1020
cagctgccca taaccgtgga ttgacaggtt ctggtgtaaa agttgctgtc ctcgatacag 1080
gtatttccac tcatccagac ttaaatattc gtggtggcgc tagctttgta ccaggggaac 1140
catccactca agatgggaat gggcatggca cgcatgtggc cgggacgatt gctgctttaa 1200
acaattcgat tggcgttctt ggcgtagcgc cgagcgcgga actatacgct gttaaagtat 1260
taggggcgag cggttcaggt tcggtcagct cgattgccca aggattggaa tgggcaggga 1320
acaatggcat gcacgttgct aatttgagtt taggaagccc ttcgccaagt gccacacttg 1380
agcaagctgt taatagcgcg acttctagag gcgttcttgt tgtagcggca tctgggaatt 1440
caggtgcagg ctcaatcagc tatccggccc gttatgcgaa cgcaatggca gtcggagcta 1500
ctgaccaaaa caacaaccgc gccagctttt cacagtatgg cgcagggctt gacattgtcg 1560
caccaggtgt aaacgtgcag agcacatacc caggttcaac gtatgccagc ttaaacggta 1620
catcgatggc tactcctcat gttgcaggtg cagcagccct tgttaaacaa aagaacccat 1680
cttggtccaa tgtacaaatc cgcaatcatc taaagaatac ggcaacgagc ttaggaagca 1740
cgaacttgta tggaagcgga cttgtcaatg cagaagcggc aacgcgtcat gctcacgcac 1800
attcagtctc acaggaagcg agcgtaacaa ggatgagttc agtggagaag aagccggagg 1860
gagtgaacac cggtgctggt gaccgtcaca acctgaagac agagtggcca gagttggtgg 1920
ggaaatcggt ggaggaggcc aagaaggtga ttctgcagga caagccagag gcgcaaatca 1980

tagttctgcc ggtggggaca attgtgacca tggaatatcg gatcgaccgc gtccgcctct 2040
ttgtcgataa actcgacaac attgcccagg tccccagggt cggctagatc cagaagcggc 2100
aacacgctaa tcaataaaaa aacgctgtgc ggttaaaggg cacagcgttt ttttgtgtat 2160
ggatcc 2166

<210> 18
<211> 2267
<212> DNA
<213> Artificial Sequence

<220>
<223> Description of Artificial sequence: construct B

<400> 18

```
gaattccggc ccaacgatgg ctgatttccg ggttgacggc cggcggaacc aaggggtgat 60
cggtcggcgg aaatgaaggc ctgcggcgag tgcgggcctt ctgttttgag gattataatc 120
agagtatatt gaaagtttcg cgatcttttc gtataattgt tttaggcata gtgcaatcga 180
ttgtttgaga aaagaagaag accataaaaa taccttgtct gtcatcagac agggtatttt 240
ttatgctgtc cagactgtcc gctgtgtaaa aataaggaat aaagggggt tgttattatt 300
ttactgatat gtaaaatata atttgtataa gaaaatgaga gggagaggaa acatgattca 360
aaaacgaaag cggacagttt cgttcagact tgtgcttatg tgcacgctgt tatttgtcag 420
tttgccgatt acaaaaacat cagccgtaaa tggcacgctg atgcagtatt ttgaatggta 480
tacgccgaac gacggccagc attggaaacg attgcagaat gatgcggaac atttatcgga 540
ttaacttaac gttaatattt gtttcccaat aggcaaatct ttctaacttt gatacgttta 600
aactaccagc ttggacaagt tggtataaaa atgaggaggg aaaccgaatg aagaaaccgt 660
tggggaaaat tgtcgcaagc accgcactac tcatttctgt tgcttttagt tcatcgatcg 720
catcggctgc tgaagaagca aaagaaaaat atttaattgg ctttaatgag caggaagctg 780
tcagtgagtt tgtagaacaa gtagaggcaa atgacgaggt cgccattctc tctgaggaag 840
aggaagtcga aattgaattg cttcatgaat ttgaaacgat tcctgtttta tccgttgagt 900
taagcccaga agatgtggac gcgcttgaac tcgatccagc gatttcttat attgaagagg 960
atgcagaagt aacgacaatg gcgcaatcag tgccatgggg aattagccgt gtgcaagccc 1020
cagctgccca taaccgtgga ttgacaggtt ctggtgtaaa agttgctgtc ctcgatacag 1080
gtatttccac tcatccagac ttaaatattc gtggtggcgc tagctttgta ccaggggaac 1140
catccactca agatgggaat gggcatggca cgcatgtggc cgggacgatt gctgctttaa 1200
acaattcgat tggcgttctt ggcgtagcgc cgagcgcgga actatacgct gttaaagtat 1260
taggggcgag cggttcaggt tcggtcagct cgattgccca aggattggaa tgggcaggga 1320
acaatggcat gcacgttgct aatttgagtt taggaagccc ttcgccaagt gccacacttg 1380
agcaagctgt taatagcgcg acttctagag gcgttcttgt tgtagcggca tctgggaatt 1440
caggtgcagg ctcaatcagc tatccggccc gttatgcgaa cgcaatggca gtcggagcta 1500
ctgaccaaaa caacaaccgc gccagctttt cacagtatgg cgcagggctt gacattgtcg 1560
caccaggtgt aaacgtgcag agcacatacc caggtcaacg tatgccagct taaacggtac 1620
atcgatggct actcctcatg ttgcaggtgc agcagccctt gttaaacaaa agaacccatc 1680
ttggtccaat gtacaaatcc gcaatcatct aaagaatacg gcaacgagct taggaagcac 1740
gaacttgtat ggaagcggac ttgtcaatgc agaagcggca acgcgttaaa aattcggaat 1800
atttatacaa tatcatatgt tacacattga aaggggagga gaatcatgaa acaacaaaaa 1860
cggctttacg cccgattgct gacgctgtta tttgcgctca tcttcttgct gcctcattct 1920
gcagcagcgg cggcaagttc agtggagaag aagccggagg gagtgaacac cggtgctggt 1980
gaccgtcaca acctgaagac agagtggcca gagttggtgg ggaaatcggt ggaggaggcc 2040
aagaaggtga ttctgcagga caagccagag gcgcaaatca tagttctgcc ggtggggaca 2100
attgtgacca tggaatatcg gatcgaccgc gtccgcctct ttgtcgataa actcgacaac 2160
attgcccagg tccccagggt cggctagatc ccagaagcgg caacacgcta atcaataaaa 2220
aaacgctgtg cggttaaagg gcacagcgtt tttttgtgta tggatcc 2267
```

<210> 19

<211> 1575

<212> DNA

<213> Artificial Sequence

<220>

<223> Description of Artificial Sequence: construct c

<400> 19

```
ttaaatattc gtggtggcgc tagctttgta ccaggggaac catccactca agatgggaat 60
gggcatggca cgcatgtggc cgggacgatt gctgctttaa acaattcgat tggcgttctt 120
ggcgtagcgc cgagcgcgga actatacgct gttaaagtat taggggcgag cggttcaggt 180
tcggtcagct cgattgccca aggattggaa tgggcaggga acaatggcat gcacgttgct 240
```

```
aatttgagtt taggaagccc ttcgccaagt gccacacttg agcaagctgt taatagcgcg 300
acttctagag gcgttcttgt tgtagcggca tctgggaatt caggtgcagg ctcaatcagc 360
tatccggccc gttatgcgaa cgcaatggca gtcggagcta ctgaccaaaa caacaaccgc 420
gccagctttt cacagtatgg cgcagggctt gacattgtcg caccaggtgt aaacgtgcag 480
agcacatacc caggttcaac gtatgccagc ttaaacggta catcgatggc tactcctcat 540
gttgcaggtg cagcagccct tgttaaacaa aagaacccat cttggtccaa tgtacaaatc 600
cgcaatcatc taaagaatac ggcaacgagc ttaggaagca cgaacttgta tggaagcgga 660
cttgtcaatg cagaagcggc aacacgctaa tcaataaaaa aacgctgtgc ggttaaaggg 720
cacagcgttt ttttgtgtat gaatcgaaaa agagaacaga tcgcaggtct caaaaatcga 780
gcgtaaaggg ctgtttaaag ctctttacgc tcgcaggtct tatcgctata caatggaaaa 840
ttcacgtctt ttgactttca tggcatattt atttaagtat tcgtttgctt tttcgtactc 900
tccgtttttc tggtaccatt gcgccagctc aattgcatag tggactggtt cttctttatt 960
atcaagcttn ctgcagngtc gacnggatcc gtcgacggcc gcagatgctg ctgaagagat 1020
tattaaaaag ctgaaagcaa aaggctatca attggtaact gtatctcagc ttgaagaagt 1080
gaagaagcag agaggctatt gaataaatga gtagaagcgc catatcggcg cttttctttt 1140
ggaagaaaat atagggaaaa tggtacttgt taaaaattcg gaatatttat acaatatcat 1200
atgttacaca ttgaaagggg aggagaatca tgaaacaaca aaaacggctt tacgcccgat 1260
tgctgacgct gttatttgcg ctcatcttct tgctgcctca ttctgcagca gcggcggcaa 1320
gttcagtgga gaagaagccg gagggagtga acaccggtgc tggtgaccgt cacaacctga 1380
agacagagtg gccagagttg gtggggaaat cggtggagga ggccaagaag gtgattctgc 1440
aggacaagcc agaggcgcaa atcatagttc tgccggtggg gacaattgtg accatggaat 1500
atcggatcga ccgcgtccgc ctctttgtcg ataaactcga caacattgcc caggtcccca 1560
gggtcggcta gatct                                                  1575
```

<210> 20
<211> 2588
<212> DNA
<213> Artificial Sequence

<220>
<223> Description of Artificial Sequence: Construct D

<400> 20

```
gaattccggc ccaacgatgg ctgatttccg ggttgacggc cggcggaacc aaggggtgat 60
cggtcggcgg aaatgaaggc ctgcggcgag tgcgggcctt ctgttttgag gattataatc 120
agagtatatt gaaagtttcg cgatcttttc gtataattgt tttaggcata gtgcaatcga 180
ttgtttgaga aaagaagaag accataaaaa taccttgtct gtcatcagac agggtatttt 240
ttatgctgtc cagactgtcc gctgtgtaaa aataaggaat aaagggggt tgttattatt 300
ttactgatat gtaaaatata atttgtataa gaaaatgaga gggagaggaa acatgattca 360
aaaacgaaag cggacagttt cgttcagact tgtgcttatg tgcacgctgt tatttgtcag 420
tttgccgatt acaaaaacat cagccgtaaa tggcacgctg atgcagtatt ttgaatggta 480
tacgccgaac gacggccagc attggaaacg attgcagaat gatgcggaac atttatcgga 540
ttaacttaac gttaatattt gtttcccaat aggcaaatct ttctaacttt gatacgttta 600
aactaccagc ttggacaagt tggtataaaa atgaggaggg aaaccgaatg aagaaaccgt 660
tggggaaaat tgtcgcaagc accgcactac tcatttctgt tgcttttagt tcatcgatcg 720
catcggctgc tgaagaagca aaagaaaaat atttaattgg ctttaatgag caggaagctg 780
tcagtgagtt tgtagaacaa gtagaggcaa atgacgaggt cgccattctc tctgaggaag 840
aggaagtcga aattgaattg cttcatgaat ttgaaacgat tcctgtttta tccgttgagt 900
taagcccaga agatgtggac gcgcttgaac tcgatccagc gatttcttat attgaagagg 960
atgcagaagt aacgacaatg gcgcaatcag tgccatgggg aattagccgt gtgcaagccc 1020
cagctgccca taaccgtgga ttgacaggtt ctggtgtaaa agttgctgtc ctcgatacag 1080
gtatttccac tcatccagac ttaaatattc gtggtggcgc tagctttgta ccaggggaac 1140
catccactca agatgggaat gggcatggca cgcatgtggc cgggacgatt gctgctttaa 1200
acaattcgat tggcgttctt ggcgtagcgc cgagcgcgga actatacgct gttaaagtat 1260
taggggcgag cggttcaggt tcggtcagct cgattgccca aggattggaa tgggcaggga 1320
acaatggcat gcacgttgct aatttgagtt taggaagccc ttcgccaagt gccacacttg 1380
agcaagctgt taatagcgcg acttctagag gcgttcttgt tgtagcggca tctgggaatt 1440
caggtgcagg ctcaatcagc tatccggccc gttatgcgaa cgcaatggca gtcggagcta 1500
ctgaccaaaa caacaaccgc gccagctttt cacagtatgg cgcagggctt gacattgtcg 1560
caccaggtgt aaacgtgcag agcacatacc caggttcaac gtatgccagc ttaaacggta 1620
catcgatggc tactcctcat gttgcaggtg cagcagccct tgttaaacaa aagaacccat 1680
cttggtccaa tgtacaaatc cgcaatcatc taaagaatac ggcaacgagc ttaggaagca 1740
cgaacttgta tggaagcgga cttgtcaatg cagaagcggc aacacgctaa tcaataaaaa 1800
aacgctgtgc ggttaaaggg cacagcgttt ttttgtgtat gaatcgaaaa agagaacaga 1860
tcgcaggtct caaaaatcga gcgtaaaggg ctgtttaaag ctctttacgc tcgcaggtct 1920
tatcgctata caatggaaaa ttcacgtctt ttgactttca tggcatattt atttaagtat 1980
tcgtttgctt tttcgtactc tccgtttttc tggtaccatt gcgccagctc aattgcatag 2040

tggactggtt cttctttatt atcaagcttn ctgcagngtc gacnggatcc gtcgacggcc 2100
gcagatgctg ctgaagagat tattaaaaag ctgaaagcaa aaggctatca attggtaact 2160
gtatctcagc ttgaagaagt gaagaagcag agaggctatt gaataaatga gtagaagcgc 2220
catatcggcg cttttctttt ggaagaaaat atagggaaaa tggtacttgt taaaaattcg 2280
gaatatttat acaatatcat atgttacaca ttgaaagggg aggagaatca tgagttcagt 2340
ggagaagaag ccggagggag tgaacaccgg tgctggtgac cgtcacaacc tgaagacaga 2400
gtggccagag ttggtgggga aatcggtgga ggaggccaag aaggtgattc tgcaggacaa 2460
gccagaggcg caaatcatag ttctgccggt ggggacaatt gtgaccatgg aatatcggat 2520
cgaccgcgtc cgcctctttg tcgataaact cgacaacatt gcccaggtcc ccagggtcgg 2580
ctagatct                                                          2588
```